# EUROPEAN PATENT SPECIFICATION

(11) **EP 0 710 285 B1**
(45) Date of publication and mention of the grant of the patent: **11.04.2001**
(21) Application number: 94920953.0
(22) Date of filing: 17.06.1994
(51) Int. Cl.: C12N 15/15, C07K 14/815, C07K 19/00, C12N 15/62, A61K 38/58, C12N 15/70, C12N 15/85, C12N 1/21, C12N 5/16

(54) **ANALOGUES OF AN ANTI-THROMBIN POLYPEPTIDE AND PROCESS FOR THEIR PREPARATION**
ANALOGE EINES ANTITHROMBOTISCHEN POLYPEPTIDES UND VERFAHREN ZU IHRER HERESTELLUNG
ANALOGUES D'UN POLYPEPTIDE ANTITHROMBINE ET LEUR PROCEDE DE PREPARATION

(30) Priority: 22.07.1993 IT MI931627
(43) Date of publication of application: 08.05.1996
(73) Proprietor: MINISTERO DELL' UNIVERSITA' E DELLA RICERCA SCIENTIFICA E TECNOLOGICA, 00196 Roma (IT)
(72) Inventor: SCACHERI, Emanuela, I-20025 Legnano (IT); VISCO, Carlo, I-20156 Milan (IT); MOLINARI, Antonio, I-20125 Milan (IT); GERNA, Marco, I-20161 Milan (IT); BOLIS, Giorgio, I-20015 Parabiago (IT)
(74) Representative: Frignoli, Luigi
(86) International application number: EP9401979
(87) International publication number: WO9503409

(56) References cited:
- EP-A- 0 373 767
- EP-A- 0 501 821
- EP-A- 0 520 502
- EUR. J. BIOCHEM. (1993), CODEN: EJBCAI;ISSN: 0014-2956, vol. 214, no.1, May 1993 pages 295-304, SCACHERI, EMANUELA ET AL 'Novel hirudin variants from the leech Hirudinaria manillensis. Amino acid sequence, cDNA cloning and genomic organization'

### SEQUENCE LISTING

(1) INFORMATION FOR SEQ ID NO:1:
   (i) SEQUENCE CHARACTERISTICS:
      (A) LENGTH: 64 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (xi) SEQUENCE DESCRIPTION: SEQ ID NO: 1:
   Val Ser Tyr Thr Asp Cys Thr Glu Ser Gly Gln Asn Tyr Cys Leu Cys
   1               5                   10                  15
   Val Gly Gly Asn Leu Cys Gly Gly Gly Lys His Cys Glu Met Asp Gly
               20                  25                  30
   Ser Gly Asn Lys Cys Val Asp Gly Glu Gly Thr Pro Lys Pro Lys Ser
           35                  40                  45
   Gln Thr Glu Gly Asp Phe Glu Glu Ile Pro Asp Glu Asp Ile Leu Asn
       50                  55                  60
(2) INFORMATION FOR SEQ ID NO: 2:
   (i) SEQUENCE CHARACTERISTICS:
      (A) LENGTH: 64 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (xi) SEQUENCE DESCRIPTION: SEQ ID NO: 2:
   Val Ser Tyr Thr Asp Cys Thr Glu Ser Gly Gln Asn Tyr Cys Leu Cys
   1               5                   10                  15
   Val Gly Ser Asn Val Cys Gly Glu Gly Lys Asn Cys Gln Leu Ser Ser
               20                  25                  30
   Ser Gly Asn Gln Cys Val His Gly Glu Gly Thr Pro Lys Pro Lys Ser
           35                  40                  45
   Gln Thr Glu Gly Asp Phe Glu Glu Ile Pro Asp Glu Asp Ile Leu Asn
       50                  55                  60
(3) INFORMATION FOR SEQ ID NO:3:
   (i) SEQUENCE CHARACTERISTICS:
      (A) LENGTH: 42 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (xi) SEQUENCE DESCRIPTION: SEQ ID NO:3:
   Val Ser Tyr Thr Asp Cys Thr Glu Ser Gly Gln Asn Tyr Cys Leu Cys
   1               5                   10                  15
   Val Gly Ser Asn Val Cys Gly Glu Gly Lys Asn Cys Gln Leu Ser Ser
               20                  25                  30
   Ser Gly Asn Gln Cys Val His Gly Glu Gly
           35                  40
(4) INFORMATION FOR SEQ ID NO:4:
   (i) SEQUENCE CHARACTERISTICS:
      (A) LENGTH: 63 base pairs
      (B) TYPE: nucleic acid
      (C) STRANDEDNESS: double
      (D) TOPOLOGY: linear
   (ii) MOLECULE TYPE: DNA
   (vi) ORIGINAL SOURCE:
      (A) ORGANISM: E. coli
   (ix) FEATURE:
      (A) NAME/KEY: misc_feature
      (B) LOCATION: 1..63
      (D) OTHER INFORMATION: /function= "leader peptide" /standard_name= "OmpA leader"
   (xi) SEQUENCE DESCRIPTION: SEQ ID N0:4:
(5) INFORMATION FOR SEQ ID NO:5:
   (i) SEQUENCE CHARACTERISTICS:
      (A) LENGTH: 48 base pairs
      (B) TYPE: nucleic acid
      (C) STRANDEDNESS: double
      (D) TOPOLOGY: linear
   (ii) MOLECULE TYPE: DNA
   (vi) ORIGINAL SOURCE:
      (A) ORGANISM: vesicular stomatitis virus
   (ix) FEATURE:
      (A) NAME/KEY: misc_feature
      (B) LOCATION: 1..48
      (D) OTHER INFORMATION: /function= "leader peptide" /standard_name= "VSV G protein leader"
   (xi) SEQUENCE DESCRIPTION: SEQ ID NO:5:
   ATGAAGTGCC TTTTGTACTT AGCCTTTTTA TTCATTGGGG TGAATTGC 48
(6) INFORMATION FOR SEQ ID NO:6:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (B) LOCATION: 1..13
      (D) OTHER INFORMATION: /note= "This sequence is amino acids 1..13 of seq id no:1"
   (xi) SEQUENCE DESCRIPTION: SEQ ID NO:6:
   Val Ser Tyr Thr Asp Cys Thr Glu Ser Gly Gln Asn Tyr
   1               5                   10
(7) INFORMATION FOR SEQ ID NO:7:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (B) LOCATION: 1..13
      (D) OTHER INFORMATION: /note= "This sequence is amino acids 14..26 of seq id no:1"
   (xi) SEQUENCE DESCRIPTION: SEQ ID NO:7:
   Cys Leu Cys Val Gly Gly Asn Leu Cys Gly Gly Gly Lys
   1               5                   10
(8) INFORMATION FOR SEQ ID NO:8:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (B) LOCATION: 1..10
      (D) OTHER INFORMATION: /note= "This sequence is amino acids 27..36 of seq id no:1"
   (xi) SEQUENCE DESCRIPTION: SEQ ID NO:8:
   His Cys Glu Met Asp Gly Ser Gly Asn Lys
   1               5                   10
(9) INFORMATION FOR SEQ ID NO:9:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (B) LOCATION: 1..11
      (D) OTHER INFORMATION: /note= "This sequence is amino acids 37..47 of seq id no:1"
   (xi) SEQUENCE DESCRIPTION: SEQ ID NO:9:
   Cys Val Asp Gly Glu Gly Thr Pro Lys Pro Lys
   1               5                   10
(10) INFORMATION FOR SEQ ID NO: 10:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (B) LOCATION: 1..11
      (D) OTHER INFORMATION: /note= "This sequence is amino acids 37..47 of seq id no:1"
   (xi) SEQUENCE DESCRIPTION: SEQ ID NO: 10:
   Cys Val Asp Gly Glu Gly Xaa Pro Lys Pro Lys
   1               5                   10
(11) INFORMATION FOR SEQ ID NO: 11:
   (i) SEQUENCE CHARACTERISTICS:
      (A) LENGTH: 17 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (A) NAME/KEY: Region
      (B) LOCATION: 1..16
      (D) OTHER INFORMATION: /note= "amino acids 48..64 of seq id no:1"
   (xi) SEQUENCE DESCRIPTION: SEQ ID NO:11:
   Ser Gln Thr Glu Gly Asp Phe Glu Glu Ile Pro Asp Glu Asp Ile Leu
   1 5 10 15
   Asn
(12) INFORMATION FOR SEQ ID NO: 12:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (B) LOCATION: 1..13
      (D) OTHER INFORMATION: /note= "This sequence is amino acids 1..13 of seq id no:2"
   (xi) SEQUENCE DESCRIPTION: SEQ ID NO: 12:
   Val Ser Tyr Thr Asp Cys Thr Glu Ser Gly Gln Asn Tyr
   1 5 10
(13) INFORMATION FOR SEQ ID NO:13:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (B) LOCATION: 1..13
      (D) OTHER INFORMATION: /note= "This sequence is amino acids 14..26 of seq id no:2"
   (xi) SEQUENCE DESCRIPTION: SEQ ID NO: 13:
   Cys Leu Cys Val Gly Ser Asn Val Cys Gly Glu Gly Lys
   1 5 10
(14) INFORMATION FOR SEQ ID NO:14:
   (i) SEQUENCE CHARACTERISTICS:
      (A) LENGTH: 21 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (A) NAME/KEY: Region
      (B) LOCATION: 1..21
      (D) OTHER INFORMATION: /note= "This sequence is amino acids 27..47 of seq id no:2"
   (xi) SEQUENCE DESCRIPTION: SEQ ID NO: 14:
   Asn Cys Gln Leu Ser Ser Ser Gly Asn Gln Cys Val His Gly Glu Gly
   1               5                   10                  15
   Xaa Pro Lys Pro Lys
               20
(15) INFORMATION FOR SEQ ID NO:15:
   (i) SEQUENCE CHARACTERISTICS:
      (A) LENGTH: 17 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (ix) FEATURE:
      (A) NAME/KEY: Region
      (B) LOCATION: 1..21
      (D) OTHER INFORMATION: /note= "This sequence is amino acids 48..64 of seq id no:2"
   (xi) SEQUENCE DESCRIPTION: SEQ ID NO:15:
   Ser Gln Thr Glu Gly Asp Phe Glu Glu Ile Pro Asp Glu Asp Ile Leu
   1               5                   10                  15
   Asn
(16) INFORMATION FOR SEQ ID NO:16:
   (i) SEQUENCE CHARACTERISTICS:
      (A) LENGTH: 198 base pairs
      (B) TYPE: nucleic acid
      (C) STRANDEDNESS: double
      (D) TOPOLOGY: linear
   (ii) MOLECULE TYPE: DNA
   (vi) ORIGINAL SOURCE:
      (A) ORGANISM: Hirudinaria manillensis
   (xi) SEQUENCE DESCRIPTION: SEQ ID NO:16:
   GTTTCTTACA CCGACTGCAC CGAATCTGGC CAGAACTACT GCCTGTGCGT 50
   TGGTTCTAAC GTTTGCGGTG AAGGTAAAAA CTGCCAGCTG TCTTCTTCTG 100
   GTAACCAGTG CGTTCACGGT GAAGGTACCC CGAAACCGAA ATCTCAGACT 150
   GAAGGTGACT TCGAAGAAAT TCCGGACGAA GACATCCTGG TTGGTTAG 198
(18) INFORMATION FOR SEQ ID NO:18:
   (i) SEQUENCE CHARACTERISTICS:
      (A) LENGTH: 116 base pairs
      (B) TYPE: nucleic acid
      (C) STRANDEDNESS: single
      (D) TOPOLOGY: linear
   (ii) MOLECULE TYPE: DNA
   (vii) IMMEDIATE SOURCE:
      (B) CLONE: oligo 1
   (xi) SEQUENCE DESCRIPTION: SEQ ID NO:18:
   AGCTTTGATA ACGAGGCGCA AAAAATGAAA AAGACAGCTA TCGCGATTGC 50
   AGTGGCACTG GCTGGTTTCG CTACCGTAGC GCAGGCCGTT TCTTACACCG 100
   ACTGCACCGA ATCTGG 116
(19) INFORMATION FOR SEQ ID NO:19:
   (i) SEQUENCE CHARACTERISTICS:
      (A) LENGTH: 117 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: Hirudinaria manillensis
   (vii) IMMEDIATE SOURCE:
      (B) CLONE: oligo 2
   (xi) SEQUENCE DESCRIPTION: SEQ ID NO: 19:
   AACTATTGCT CCGCGTTTTT TACTTTTTCT GTCGATAGCG CTAACGTCAC 50
   CGTGACCGAC CAAAGCGGTG GCATCGCGTC CGGCAAAGAA TGTGGCTGAC 100
   GTGGCTTAGA CCGGTCT 117
(20) INFORMATION FOR SEQ ID NO:20:
   (i) SEQUENCE CHARACTERISTICS:
      (A) LENGTH: 88 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: Hirudinaria manillensis
   (vii) IMMEDIATE SOURCE:
      (B) CLONE: oligo 3
   (xi) SEQUENCE DESCRIPTION: SEQ ID NO:20:
   CCAGAACTAC TGCCTGTGCG TTGGTTCTAA CGTTTGCGGT GAAGGTAAAA 50
   ACTGCCAGCT GTCTTCTTCT GGTAACCAGT GCGTTCAC 88
(21) INFORMATION FOR SEQ ID NO:21:
   (i) SEQUENCE CHARACTERISTICS:
      (A) LENGTH: 87 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: Hirudinaria manillensis
   (vii) IMMEDIATE SOURCE:
      (B) CLONE: oligo 4
   (xi) SEQUENCE DESCRIPTION: SEQ ID NO:21:
   TGATGACGGA CACGCAACCA AGATTGCAAA CGCCACTTCC ATTTTTGACG 50
   GTCGACAGAA GAAGACCATT GGTCACGCAA GTGCCAC 87
(22) INFORMATION FOR SEQ ID NO:22:
   (i) SEQUENCE CHARACTERISTICS:
      (A) LENGTH: 85 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: Hirudinaria manillensis
   (vii) IMMEDIATE SOURCE:
      (B) CLONE: oligo 5
   (xi) SEQUENCE DESCRIPTION: SEQ ID NO:22:
   GGTGAAGGTA CCCCGAAACC GAAATCTCAG ACTGAAGGTG ACTTCGAAGA 50
   AATTCCGGAC GAAGACATCC TGGTTGGTTA GTAAG 85
(23) INFORMATION FOR SEQ ID NO:23:
   (i) SEQUENCE CHARACTERISTICS:
      (A) LENGTH: 85 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: Hirudinaria manillensis
   (vii) IMMEDIATE SOURCE:
      (B) CLONE: oligo 6
   (xi) SEQUENCE DESCRIPTION: SEQ ID NO:23:
   TTCCATGGGG CTTTGGCTTT AGAGTCTGAC TTCCACTGAA GCTTCTTTAA 50
   GGCCTGCTTC TGTAGGACCA ACCAATCATT CCTAG 85
(24) INFORMATION FOR SEQ ID NO:24:
   (i) SEQUENCE CHARACTERISTICS:
      (A) LENGTH: 91 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: vescicular stomatitis virus
   (vii) IMMEDIATE SOURCE:
      (B) CLONE: oligo 1 VSV
   (xi) SEQUENCE DESCRIPTION: SEQ ID NO:24:
   AGCTTGGATC CACTATGAAG TGCCTTTTGT ACTTAGCCTT TTTATTCATT 50
   GGGGTGAATT GCGTTTCTTA CACCGACTGC ACCGAATCTG G 91
(25) INFORMATION FOR SEQ ID NO:25:
   (i) SEQUENCE CHARACTERISTICS:
      (A) LENGTH: 87 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: vescicular stomatitis virus
   (vii) IMMEDIATE SOURCE:
      (B) CLONE: oligo 2 VSV
   (xi) SEQUENCE DESCRIPTION: SEQ ID NO:25:
   CCAGATTCGG TGCAGTCGGT GTAAGAAACG CAATTGACCC CAATGAATAA 50
   AAAGGCTAAG TACAAAAGGC ACTTCATAGT GGATCCA 87
(26) INFORMATION FOR SEQ ID NO:26:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: Hirudinaria manillensis
   (vii) IMMEDIATE SOURCE:
      (B) CLONE: oligo 1 fusion
   (xi) SEQUENCE DESCRIPTION: SEQ ID NO:26:
   GATCCATGGT TTCTTACACC GACTGCACCG AATCTGG 37
(27) INFORMATION FOR SEQ ID NO:27:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: Hirudinaria manillensis
   (vii) IMMEDIATE SOURCE:
      (B) CLONE: oligo 2 fusion
   (xi) SEQUENCE DESCRIPTION: SEQ ID NO:27:
   CCAGATTCGG TGCAGTCGGT GTAAGAAACC ATG 33
(28) INFORMATION FOR SEQ ID NO:28:
   (i) SEQUENCE CHARACTERISTICS:
      (A) LENGTH: 150 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: baculovirus
   (vii) IMMEDIATE SOURCE:
      (B) CLONE: oligo 1 polyhedrin
   (xi) SEQUENCE DESCRIPTION: SEQ ID NO:28:
   ATCATGGAGA TAATTAAAAT GATAACCATC TCGCAAATAA ATAAGTATTT 50
   TACTGTTTTC GTAACAGTTT TGTAATAAAA AAACCTATAA ATATGCCGGA 100
   TTATTCATAC CGTCCCACCA TCGGGCGTAC CTACGTGTAC GACAACACCG 150
(29) INFORMATION FOR SEQ ID NO:29:
   (i) SEQUENCE CHARACTERISTICS:
      (A) LENGTH: 154 base pairs
      (B) TYPE: nucleic acid
      (C) STRANDEDNESS: single
      (D) TOPOLOGY: linear
   (ii) MOLECULE TYPE: DNA
   (vi) ORIGINAL SOURCE:
      (A) ORGANISM: baculovirus
   (vii) IMMEDIATE SOURCE:
      (B) CLONE: oligo 2 polyhedrin
   (xi) SEQUENCE DESCRIPTION: SEQ ID NO:29:
   GATCCGGTGT TGTCGTACAC GTAGGTACGC CCGATGGTGG GACGGTATGA 50
   ATAATCCGGC ATATTTATAG GTTTTTTTAT TACAAAACTG TTACGAAAAC 100
   AGTAGAATAC TTATTTATTT GCGAGATGGT TATCATTTTA ATTATCTCCA 150
   TGAT 154
32) INFORMATION FOR SEQ ID NO:32:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Hirudinaria manillensis
   (xi) SEQUENCE DESCRIPTION: SEQ ID NO:32:
   Met Phe Ser Leu Lys Leu Phe Val Val Phe Leu Ala Val Cys Ile Cys
   1               5                   10                  15
   Val Ser Gln Ala
               20

## Description

The present invention relates to analogues of anti-thrombin polypeptides and to a process for their preparation. The analogues have been obtained by C-terminal modification of anti-thrombin polypeptides from the leech Hirudinaria manillensis and have an improved biological activity in comparison to the polypeptides from which they are derived.

The most popular anticoagulant peptides are probably those belonging to the family of hirudins. Hirudin, originally isolated from the medicinal leech, Hirudo medicinalis, is a well known and well characterized polypeptidic inhibitor of thrombin^{1.2}. More particularly, it binds thrombin by ionic interactions thus preventing the cleavage of fibrinogen to fibrin and the subsequent fibrin-clot formation. In animal studies hirudin has demonstrated efficacy in preventing venous thrombosis, vascular shunt occlusion and thrombin-induced disseminated intravascular coagulation. In addition, hirudin exhibits low toxicity, little or no antigenicity and a very short clearance time from circulation.³

Polypeptides with anticoagulant properties have been isolated from a different leech species, Hirudinaria manillensis (EP-A-0347376 and WO 90/05143). This leech is evolutionarily more advanced than Hirudo medicinalis and could therefore synthesize anticoagulant peptides whose amino acid sequences may be different from those of hirudin and other known hirudin variants.

We have analysed a preparation obtained from Hirudinaria manillensis leeches. We have found three polypeptides having anti-thrombin activity, characterized by the following amino acid sequences (European Patent Application No. 92301721.4):

Amino acid residues are presented according to the three letter code (Eur. J. Biochem. 138, 9-37, 1984).

The invention provides a polypeptide comprising a sequence represented by formula (I) wherein P₁₋₆₃ is the amino acid sequence from position 1 to position 63 of the polypeptide P2 (SEQ ID NO: 2), Xaa is Val and Z is -Gly-OH and a pharmaceutically acceptable salt thereof.
The pharmaceutically acceptable salts of the polypeptides may be acid addition salts. They may be salts with an inorganic acid such as a hydrohalic acid, such as hydrochloric acid; sulphuric acid; phosphoric acid; or pyrophosphoric acid. The salts may be salts with an organic acid such as benzenesulphonic, p-toluenesulphonic, methanesulphonic, acetic, lactic, palmitic, stearic, malic, tartaric, ascorbic or citric acid. The analogues also contain free carboxyl groups and may therefore be present as sodium, calcium, potassium, magnesium or ammonium salts or salts with a physiologically tolerable organic nitrogen-containing base. The analogues can also be in the form of inner salts.

The polypeptides of the invention are able to inhibit the proteolytic effects of thrombin, for example fibrin formation from fibrinogen and the activation of factor V and factor VIII. The polypeptides are therefore useful, for example, as anticoagulant and antithrombotic drugs, for the treatment of thromboembolic pathologies such as disseminate intravascular coagulation, cerebral embolisms, deep venous thrombosis and arterial thrombosis. They are also useful in prophylactic treatment of coagulation disorders, for example for the therapy of acute myocardial infarction.

The polypeptides of the invention consist essentially of the sequence P2₁₋₆₃-Val-Gly-OH. However, the polypeptides may be preceded by all or part, typically a C-terminal part, of a leader sequence. The leader sequence may be a native leader sequence of a natural polypeptide from which the polypeptides of the invention are derived, or may be a foreign leader sequence. The leader sequence may be capable of directing secretion of the polypeptides of the invention. Two of the natural anti-thrombin polypeptides are expressed with a leader sequence which is cleaved subsequently. All or part of this leader sequence may therefore be present in the polypeptide of the invention, the sequence being:

A polypeptide according to the invention, or a salt thereof, may be prepared by: 1) isolating an anti-thrombin polypeptide from the tissue or secretions of a leech of the species Hirudinaria manillensis and 2) subsequently modifying the C-terminal end of such a polypeptide. More specifically, the polypeptide can be obtained by obtaining a preparation of leech extract, subjecting the preparation to high pressure liquid chromatography, and modifying the C-terminal end of the polypeptide.

A polypeptide according to the invention or a salt thereof can then be prepared by:
(a) providing a non-human host, transformed with an expression vector comprising a DNA sequence encoding a said polypeptide, under such conditions that the said polypeptide is expressed;
(b) isolating the said polypeptide thus obtaining a polypeptide of the invention wherein Z is Gly-OH or a pharmaceutically acceptable salt thereof.

This approach is typically based on obtaining a nucleotide sequence encoding the polypeptide it is wished to express and expressing the polypeptide in a recombinant non-human organism. The cultivation of the genetically modified organism leads to the production of the desired product displaying biological activity. The present invention therefore further provides
- an expression vector comprising a DNA sequence encoding a polypeptide of the invention;
- a non-human host transformed with a compatible expression vector according to the invention; and
- a DNA sequence encoding a polypeptide according to the invention.

A non-human host in which a polypeptide according to the invention is able to be expressed is prepared by transforming a non-human host with a compatible expression vector of the invention. The expression vector can be prepared by:
(a) chemically synthesising a DNA sequence encoding a polypeptide of the invention; and
(b) inserting the said DNA into an expression vector.

Alternatively, an expression vector can be prepared by:
(a) producing and isolating a cDNA encoding the polypeptide P2 from mRNA of a leech of the species Hirudinaria manillensis;
(b) mutagenising the isolated cDNA so as to obtain a nucleotide sequence encoding a polypeptide of the invention; and
(c) inserting the said nucleotide sequence into an expression vector.

A polypeptide according to the invention is consequently prepared by providing a transformed non-human host under such conditions that the polypeptide is expressed. When a non-human eucaryotic host is employed, the polypeptide can be obtained glycosylated. The polypeptide can be isolated as such or in the form of a pharmaceutically acceptable salt. In this way, a polypeptide or salt according to the invention may be obtained in essentially pure form.

The polypeptides of the invention may be modified by way of amino acid extension at either or each end. A polypeptide composed of such an extended sequence must of course still exhibit anti-thrombin activity. For example, a short sequence of up to 30 amino acid residues may be provided at either or each terminus.

In order to produce a polypeptide of the invention by recombinant DNA technology, a gene encoding a said polypeptide is prepared. The invention includes DNA sequences consisting essentially of the following sequences: The DNA coding sequence typically does not include introns. The DNA sequence is isolated and purified. The gene is inserted in an expression vector able to drive production of the recombinant product. The DNA sequence may be a mutagenized cDNA sequence. The DNA sequence may be a synthetic DNA sequence. The synthetic gene is typically prepared by chemically synthesising oligonucleotides which, in total, correspond to the desired gene. The oligonucleotides are then assembled to obtain the gene.

A gene may therefore be constructed from six chemically synthesised oligonucleotides, each oligonucleotide representing about one third of one strand of a doublestranded DNA gene. The oligonucleotides are ligated and annealed to obtain the desired gene. Typically, a gene is constructed with restriction sites at each end to facilitate its subsequent manipulation.

A DNA sequence may be provided which further encodes a leader peptide as mentioned above. The leader peptide is capable of directing secretion of the polypeptide from cells in which the polypeptide is to be expressed. The sequence encoding the leader peptide is typically fused to the 5'-end of the DNA sequence encoding the polypeptide.

The leader peptide may be the OmpA leader peptide when expression in a bacterial host, such as E. coli is required. The leader peptide may be the leader peptide of vesicular stomatitis virus G protein (VSV G protein) when expression is to be in insect cells. Appropriate DNA sequences encoding the OmpA and VSV G protein leader sequences are:

A DNA sequence may be provided which encodes a fusion protein which is cleavable to release a polypeptide of the invention. A DNA sequence may be used which encodes a carrier polypeptide sequence fused via a cleavable linkage to the N-terminus of a polypeptide of the invention. The cleavable linkage may be one cleavable by cyanogen bromide.

For expression of the polypeptide, an expression vector is constructed which comprises a DNA sequence encoding the polypeptide and which is capable of expressing the polypeptide when provided in a suitable non-human host. Appropriate transcriptional and translational control elements are provided, including a promoter for the DNA sequence, a transcriptional termination site, and translational start and stop codons. The DNA sequence is provided in the correct frame such as to enable expression of the polypeptides to occur in a non-human host compatible with the vector.

The expression vector typically comprises an origin of replication and, if desired, a selectable marker gene such as an antibiotic resistance gene. A promoter is operably linked to the DNA sequence encoding the polypeptide. The expression vector may be a plasmid. In that case, preferably a promoter selected from the Pₜᵣₚ and P_{lpp/lac} promoters is operably linked to the DNA sequence. Alternatively, the expression vector may be a virus. The virus may be a recombinant baculovirus in which the polyhedrin promoter is operably linked to the DNA sequence encoding the polypeptide.

An expression vector capable of expressing the polypeptide may be prepared in any convenient fashion. A DNA fragment encoding the polypeptide may be inserted into an appropriate restriction site of an expression vector, for example a plasmid vector. A recombinant baculovirus may be prepared by:
(i) cloning a gene encoding the polypeptide into a baculovirus transfer vector at a restriction site downstream of the polyhedrin promoter; and
(ii) co-transfecting insect cells susceptible to baculovirus infection with the recombinant transfer vector from step (i) and intact wild-type baculovirus DNA.

Homologous recombination occurs, resulting in a recombinant baculovirus harbouring the polypeptide gene downstream of the polyhedrin promoter. The baculovirus transfer vector may be one having a unique cloning site downstream of the polyhedrin ATG start codon. The product that is then expressed by the resulting recombinant baculovirus will be a fusion protein in which a N-terminal portion of the polyhedrin protein is fused to the N-terminus of the polypeptide of the invention. As indicated above, a cleavable linkage may be provided at the fusion junction.

The insect cells employed in step (ii) are typically Spodoptera frugriperda cells. The wild-type baculovirus is typically Autographa californica nuclearpolyhedrosis virus (AcNPV).

An expression vector encoding the polypeptide is provided in an appropriate non-human host. Cells are transformed with the gene of the polypeptide. A transformed non-human host is provided under such conditions that the polypeptide is expressed. Transformed cells, for example, are cultivated so as to enable expression to occur. Any compatible host-vector system may be employed.

The transformed non-human host may be a prokaryotic or eukaryotic host. A bacterial or yeast host may be employed, for example E. coli or S. cerevisiae. Gram positive bacteria may be employed. A preferred bacterial host is a strain of E. coli type B. Insect cells can alternatively be used, in which case a baculovirus expression system is appropriate. The insect cells are typically Spodoptera frugiperda cells. As a further alternative, cells of a mammalian cell line may be transformed. A transgenic non-human animal, for example a non-human mammal, may be provided in which the polypeptide is produced.

The polypeptide that is expressed may be isolated and purified. A polypeptide having the aminoacid sequence depicted in formula (I) above preceded by a Met residue attributable to a translation start codon can be obtained. Alternatively, as mentioned above, a fusion protein may be obtained comprising the amino acid sequence of formula (I) fused to a carrier sequence. The carrier sequence is tipically fused to the N-terminus of the polypeptide of formula (I).
Where a suitable linkage is provided in the fusion protein between the amino acid sequence of formula (I) and the carrier sequence, a polypeptide having an amino acid sequence according to formula (I) can be released by cleavage with a suitable agent.

A polypeptide of the invention or a pharmaceutically acceptable salt thereof can also be prepared by:
(a) chemically synthesising the said polypeptide; and
(b) isolating the said polypeptide thus obtained or a pharmaceutically acceptable salt thereof.

The polypeptides can therefore be built up by chemical synthesis from single amino acids and/or preformed peptides of two or more amino acids in the order of the sequence of the desired polypeptide. Solid-phase or solution methods may be employed. The resultant polypeptide may be converted into a pharmaceutically acceptable salt if desired.

In solid-phase synthesis, the amino acid sequence of the desired polypeptide is built up sequentially from the C-terminal amino acid which is bound to an insoluble resin. When the desired polypeptide has been produced, it is cleaved from the resin. When solution-phase synthesis is employed, the desired polypeptide may again be built up from the C-terminal amino acid. The carboxy group of this acid remains blocked throughout by a suitable protecting group, which is removed at the end of the synthesis.

Whichever technique, solid-phase or solution-phase, is employed each amino acid added to the reaction system typically has a protected amino group and an activated carboxy group. Functional side-chain groups are protected too. After each step in the synthesis, the amino-protecting group is removed. Side-chain functional groups are generally removed at the end of the synthesis.

A polypeptide may be converted into a pharmaceutically acceptable salt. It may be converted into an acid addition salt with an organic or inorganic acid.
Suitable acids include acetic, succinic and hydrochloric acid. Alternatively, the polypeptide may be converted into a carboxylic acid salt such as the ammonium salt or an alkali metal salt such as the sodium or potassium salt.

A polypeptide or pharmaceutically acceptable salt thereof may be used in a pharmaceutical composition, together with a pharmaceutically acceptable carrier or excipient therefor. Such a formulation is typically for intravenous administration (in which case the carrier is generally sterile saline or water of acceptable purity). A polypeptide according to the invention is an anti-thrombin polypeptide and is suitable for treatment of thromboembolic events, such as the coagulation of blood, typically in a human patient. A polypeptide according to the present invention displays improved biological characteristics when compared to the corresponding native polypeptide from which it is derived. A polypeptide can therefore be used for the therapy and prophylaxis of thromboses and thromboembolisms, including the prophylaxis of post-operative thromboses, for acute shock therapy (for example for septic or polytraumatic shock), for the therapy of consumption coagulopathies, in haemodialyses, haemoseparations and in extracorporeal blood circulation. In one embodiment of the invention, the polypeptide or salt thereof can be coadministered with a plasminogen activator, such as tissue plasminogen activator. The dosage depends especially on the specific form of administration and on the purpose of the therapy or prophylaxis. The size of the individual doses and the administration regime cant best be determined by way of an individual judgement of the particular case of illness; the methods of determining relevant blood factors required for this purpose are familiar to the person skilled in the art. Normally, in the case of an injection the therapeutically effective amount of the compounds according to the invention is in a dosage range of from approximately 0.005 to approximately 0.1 mg/kg body weight. A range of from approximately 0.01 to approximately 0.05 mg/kg body weight is preferred. The administration is effected by intravenous, intramuscular or subcutaneous injection. Accordingly, pharmaceutical compositions for parenteral administration in single dose form contain per dose, depending on the mode of administration, from approximately 0.4 to approximately 7.5 mg of the compound according to the invention. In addition to the active ingredient these pharmaceutical compositions usually also contain a buffer, for example a phosphate buffer, which is intended to keep the pH value between approximately 3.5 and 7,-and also sodium chloride, mannitol or sorbitol for adjusting the isotonicity. They may be in freeze-dried or dissolved form, it being possible for solutions advantageously to contain an antibacterially active preservative, for example from 0.2 to 0.3% 4-hydroxybenzoic acid methyl ester or ethyl ester.

A composition for topical application can be in the form of an aqueous solution, lotion or gel, an oily solution or suspension or a fat-containing or, especially, emulsified ointment. A composition in the form of an aqueous solution is obtained, for example, by dissolving the active ingredients according to the invention, or a therapeutically acceptable salt thereof, in an aqueous buffer solution of from e.g., pH 4 to pH 6.5 and, if desired, adding a further active ingredient, for example an anti-inflammatory agent,and/or a polymeric binder, for example polyvinylpyrrolidone, and/or a preservative. The concentration of active ingredient is from approximately 0.1 to approximately 1.5mg, preferably from 0.25 to 1.0 mg, in 10 ml of a solution or 10 g of a gel.

An oily form of administration for topical application is obtained, for example, by suspending the active ingredient according to the invention, or a therapeutically acceptable salt thereof, in an oil, optionally with the addition of swelling agents, such as aluminium stearate, and/or surfactants (tensides) having an HLB value ("hydrophilic-lipophilic balance") of below 10, such as fatty acid monoesters of polyhydric alcohols, for example glycerin monostearate, sorbitan monolaurate, sorbitan monostearate or sorbitan monooleate. A fat-containing ointment is obtained, for example, by suspending the active ingredient according to the invention, or a salt thereof, in a spreadable fatty base, optionally with the addition of a tenside having an HLB value of below 10.
An emulsified ointment is obtained by triturating an aqueous solution of the active ingredient according to the invention, or a salt thereof, in a soft, spreadable fatty base with the addition of a tenside having an HLB value of below 10. All these forms for topical application can also contain preservatives. The concentration of active ingredient is from approximately 0.1 to approximately 1.5mg, preferably from 0.25 to 1.0 mg, in approximately 10 g of base.

In addition to the compositions described above and pharmaceutical compositions analogous thereto that are intended for direct medicinal use in the body of a human or a mammal, the present invention relates also to pharmaceutical compositions and preparations for medicinal use outside the living body of humans or mammals. Such compositions and preparations are used especially as anticoagulant additives to blood that is being subjected to circulation or treatment outside the body (for example extra corporeal circulation or dialysis in artificial kidneys), preservation or modification (for example haemoseparation). Such preparations, such as stock solutions or alternatively preparations in single dose form, are similar in composition to the injection preparations described above; however, the amount or concentration of active ingredient is advantageously based on the volume of blood to be treated or, more precisely, on its thrombin content. In this connection it must be borne in mind that the active ingredient according to the invention (in free form) completely deactivates approximately 5 times the amount by weight of thrombin, is physiologically harmless even in relatively large amounts, and is eliminated from the circulating blood rapidly even in high concentrations so that there is no risk of overdose, even, for example, during transfusions. Depending on the specific purpose, the suitable dose is from approximately 0.01 to approximately 1.0 mg of the active ingredient/litre of blood, although the upper limit may still be exceeded without risk. The following Examples illustrate the invention. In the accompanying drawings:

Figure 1 is a chromatogram showing the results of the HPLC analysis of Example 1. P1 to P3 denote the three different peaks obtained from the preparation according to Example 1, FT is for flow through and 4-AB is for 4-aminobenzamidine.

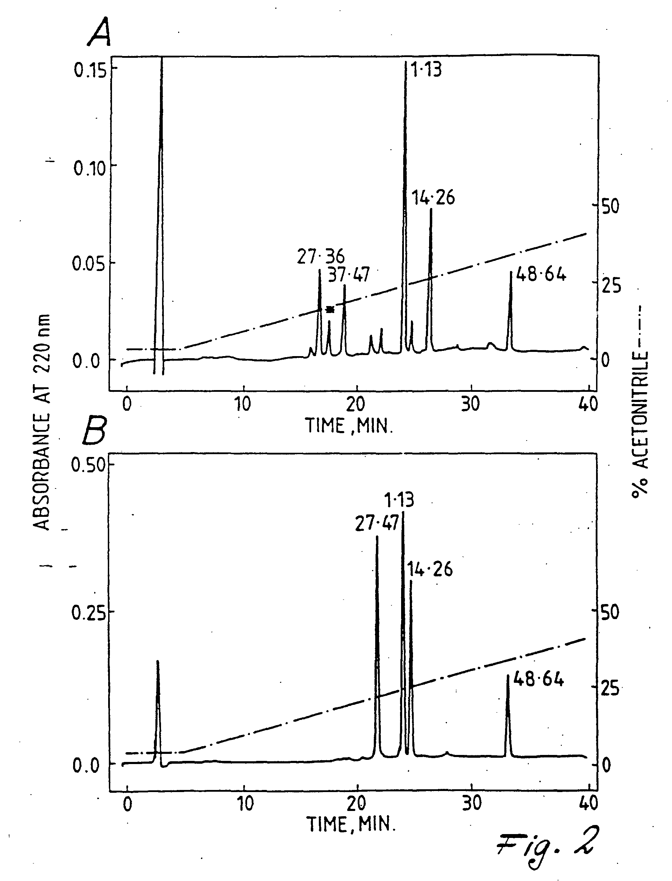

Figure 2 shows the elution profiles obtained in Example 2(b) for trypsin-digested PE-P1 (A) and PE-P2 (B).

Figure 3 shows the nucleotide sequence of the six oligos coding for the analogue P2₁₋₆₃-Val-Gly-OH linked to the OmpA leader peptide. P2₁₋₆₃-Val-Gly-OH is an analogue of Formula (I) wherein P is the anti-thrombin polypeptide P2, Xaa is Val and Z is Gly-OH. The sequence shown in bold face indicates the OmpA leader peptide; the Hind III, Bal I and BamHI restriction sites are underlined and the codons for Val₆₄ and Gly₆₅are in italics.

Figure 4 shows the scheme of the construction of the plasmid, named OMP-P2VG, which is the source of a Bal I-BamHI DNA fragment for further genetic constructions.

Figure 5 shows schematically the construction of pFC-P2VG which is the plasmid used for the production of P2₁₋₆₃-Val-Gly-OH protein in E. coli.

Figure 6 shows the general structure of the plasmid pOMPA-P2VG used for the production of P2₁₋₆₃-Val-Gly-OH in E. coli. We employed traditional gene manipulation techniques to prepare this new plasmid where the P2₁₋₆₃-Val-Gly-OH gene is under transcriptional control of the hybrid promoter P_{lpp/lac}. Even in this case, the OmpA leader peptide drives secretion of P2₁₋₆₃-Val-Gly-OH to the periplasm of E. coli.

Figure 7 shows the nucleotide sequence and assembling of the synthetic oligos used for the secretion of P2₁₋₆₃-Val-Gly-OH from insect cells. The sequence shown in bold face indicates the VSV G protein leader peptide.

Figure 8 is a schematic representation of the construction of a new recombinant M13, named VSV-P2VG, where the complete P2₁₋₆₃-Val-Gly-OH gene is linked to the VSV G protein leader peptide.

Figure 9 shows schematically the construction of pAc-P2VG which has been used as transfer vector to the baculovirus genome. pAcYM1 is the starting plasmid widely used as acceptor of heterologous sequences to be transferred to the virus.

Figure 10 shows the nucleotide sequence and assembling of the synthetic oligos coding for the beginning of the P2₁₋ ₆₃Val-Gly-OH chain. The ATG codon coding for the additional methionine residue is shown in bold face.

Figure 11 shows schematically the construction of pAcFT1, which has been used for intracellular expression. Figure 12 is a schematic representation of a new transfer plasmid, named pAcFT1-P2VG, which carries the complete P2₁₋₆₃-Val-Gly-OH sequence linked to the first 18 amino acids of polyhedrin. This plasmid has been used to transfer the heterologous sequence to the baculovirus genome.

### Example 1

An antithrombin preparation was prepared from Hirudinaria manillensis leeches according to the procedure illustrated under a) to d) below:

### a) Acetone extraction

Ethanol dried leech heads (2920 g) were finely chopped into small pieces and
treated with a mixture 40:60 acetone/water (7.5 1). After homogenisation-with stirring at room temperature, the mixture was spun for 15 min at 2,700 rpm and the supernatant was decanted; the pellet was again resuspended in 40:60 acetone:water mixture, stirred for 30 min and the mixture centrifuged for 15 min at 2,700rpm. The supernatant was pooled with the initial one and acidified to pH 4.5 with glacial acetic acid (vol. 8.5 1). The mixture was spun at 2,700 rpm for 15 minutes, then the supernatant was decanted and the pH of the solution adjusted to pH 6.0 by adding 30% ammonia. Following rotary evaporation at 35°C, the pH of the concentrated solution was lowered to 1.8; precipitated contaminants were removed by centrifuging and the raw anti-thrombin material was precipitated from the mixture using a 9-fold acetone excess. The mixture was then spun down, the pellet resuspended in acetone and again centrifuged. The precipitated material was then lyophilized.

### b) Ionic exchange purification

The raw anti-thrombin material was reconstituted in water, dialyzed against 10 mM ammonium acetate buffer at pH 4.0 and loaded onto a carboxymethyl Sepharose column (CMSepharose, Pharmacia, 2.6 x 30 cm) pre-equilibrated in the same buffer. Following a 100 ml washing with starting buffer, anti-thrombin active fractions were eluted with 20mM ammonium acetate pH 4.5, collected and pooled (1.3 1). For further purification steps, pooled fractions were concentrated to 0.5 1 in a Minitan apparatus (Millipore); the concentrated solution was neutralised with NaOH and then applied on to a Q Sepharose column equilibrated in 20 mM Tris-HCl pH 7.0. The bound material was eluted with a linear gradient of 0 - 1 M NaCl in the starting buffer. The fractions containing with anti-thrombin activity were pooled, concentrated and desalted on a Superdex S-200 column eluted with 20 mM Tris-HCl pH 7.5 at a flow rate of 4ml/min. Active pool from gel filtration was concentrated by Minitan and further purified by weak anion exchange chromatography (DEAE FPLC). The active material was loaded onto a Protein Pak DEAE-5PW column (Waters) and eluted with a gradient of 0 - 1 M NaCl in 20 mM Tris-HCl pH 6.5, at a flow rate of 1.0 ml/min. Active fractions were pooled, characterized for protein content and activity (specific activity: 800 ATU/mg), and freeze-dried in a Speed Vacconcentrator (Savant).

The thus obtained partially purified material (specific activity 800 ATU/mg) was then subjected to two additional chromatographic steps, in order to get homogenous polypeptides, as described below under c) and d):

### c) Thrombin-Sepharose

Commercial bovine thrombin (Sigma) was further purified according to the procedure described by Lundblad⁹ and then was attached to activated Sepharose CL 6B (Pharmacia) following manufacturer's instructions. The column (1.7 ml) was equilibrated with 50 mM Tris-HCl pH 8.3 and the freeze-dried material from DEAE-FPLC (reconstituted in buffer) was loaded. The column was subjected to three washings, in starting buffer, then in the same buffer containing 3.0 M NaCl and again with starting buffer (each washing was three times the column volume). Flow rate was 0.3 ml/min. The bound material was eluted with 10 ml of 0.1M 4-aminobenzamidine in 25 mM HCl. The active fractions were pooled and buffer exchanged in 50 mM Tris-HCl pH 8.3 onto a PD-10 column (Pharmacia).

Unbound material eluted from the column by washing in starting buffer and still containing anti-thrombin activity, was reloaded onto the column until all the activity was bound and chromatographed.

### d) RP-HPLC

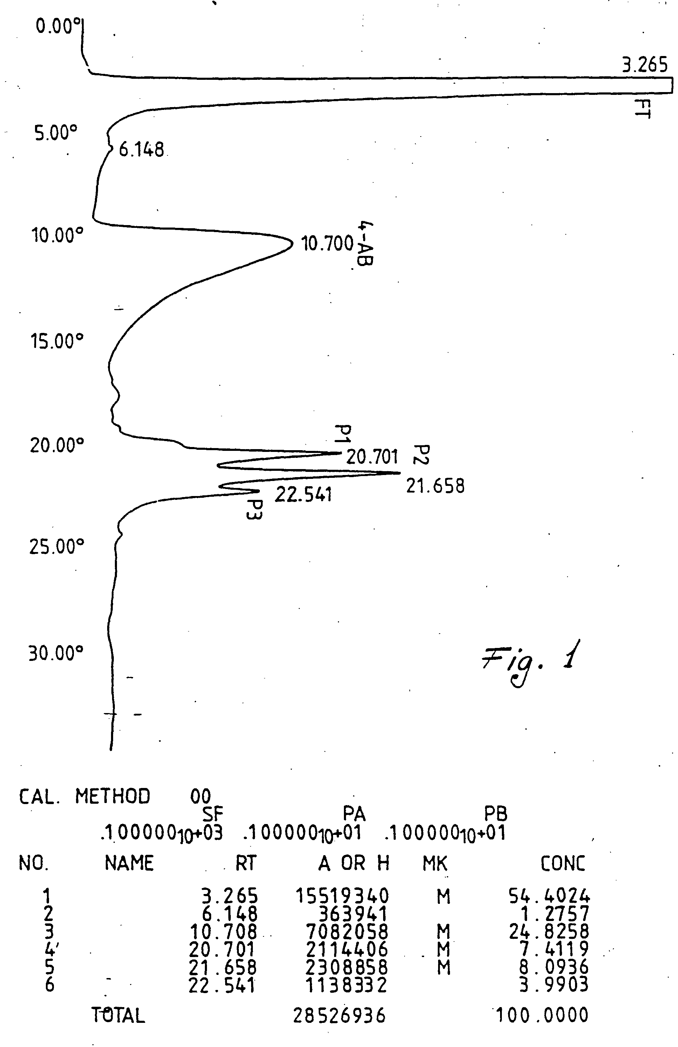

Material obtained after affinity chromatography was finally purified by reverse phase high performance chromatography (RP-HPLC) on a C4 Vydac column (4.6 x 250 mm, 5µm) using 20 mM sodium phosphate pH 7.5 as first eluent and 50% acetonitrile in water as modified. Anti-thrombin polypeptides were eluted with a linear gradient from 5% to 55% of eluent B in 45 minutes, at room temperature with a flow rate of 1.0 ml/min. The resulting chromatogram is shown in Figure 1.

Peaks of protein (detected at 220 nm) were manually collected, concentrated under vacuum and re-chromatographed under the same conditions.

Pure anti-thrombin polypeptides after C4 HPLC were characterized for protein content, amino acid composition, N-terminal sequence, C-terminal end and their activity determined by in vitro assays (ATU/NIH test and "thrombin time" test). Each of the three peaks of protein has been found to be endowed with anti-thrombin activity.

The complete amino acid sequences of the polypeptides labeled P1 and P2 in Figure 1 were determined by N-terminal sequencing of the peptides obtained from tryptic and V8 protease digests. The sequences are reported above together with the partial amino acid sequence of the other polypeptide (P3).

### Example 2 - Tryptic digestion and peptide mapping of pyridylethylated (PE) P1 and P2

a) Reduction/Alkylation- Active fractions purified by affinity chromatography on Thrombin-Sepharose (Example 1c) were pooled and buffer exchanged in 10 mM Tris-HCl pH 8.3 onto a PD-10 column. The active pool (about 50 *µ*g) was concentrated in a Speed-Vac centrifuge (Savant) and treated with 100 µl of 1% β-mercaptoethanol in 6M guanidine-HCl / 50 mM Tris-HCl pH 8.5, under nitrogen, in the dark, for 2 hours at room temperature. Then 4 µl of 4-vinyl-pyridine (neat) were added and the mixture incubated again for 2 hours as above¹⁰.
   Pyridylethylated polypeptides were first recovered from the reaction mixture by RP-HPLC on a C4 Vydac (4.6x250mm, 5µm) column eluted with a 90 min. linear gradient from 5-65% acetonitrile in 0.1% TFA, at a flow rate of 1.0ml/min. Under such conditions the mixture of anti-thrombin polypeptides is poorly resolved so that they have to be re-chromatographed on the same column using the elution system sodium phosphate/acetonitrile with the conditions already described in Example 1d.
b) Trypsin digestion and peptide mapping of PE-P1 and PE-P2 - Purified PE-P1 and PE-P2 (respectively 10 and 20 µg) were digested with TPCK-treated trypsin (Sigma) in 200 µl of 1% ammonium bicarbonate pH 8.0 in the presence of
   0.2 M sodium phosphate. Trypsin was added at an enzyme-to-substrate ratio of 1:20 (w/w) and incubation was carried out for 4 hours at 37°C. Digestion was stopped by freeze-drying in Savant.

Peptides obtained by tryptic digestion were separated on a µBondapak C18 column (3.9x300 mm, 10µ Waters) or on a C4-Vydac (4.6x250 mm, 5µm) column eluted using a 60 min. linear gradient from 5-65% acetonitrile in 0.1% TFA, at a flow rate of 1.0 ml/min. Eluted peaks were manually collected, concentrated in Savant and then subjected either to amino acid analysis and to N-terminal sequence analysis on a pulsed liquid-phase mod.477A. Sequencer (Applied Biosystems).

The results of C4-HPLC peptide mapping of trypsin-digested PE-P1 (A) and PE-P2 (B) are shown below.

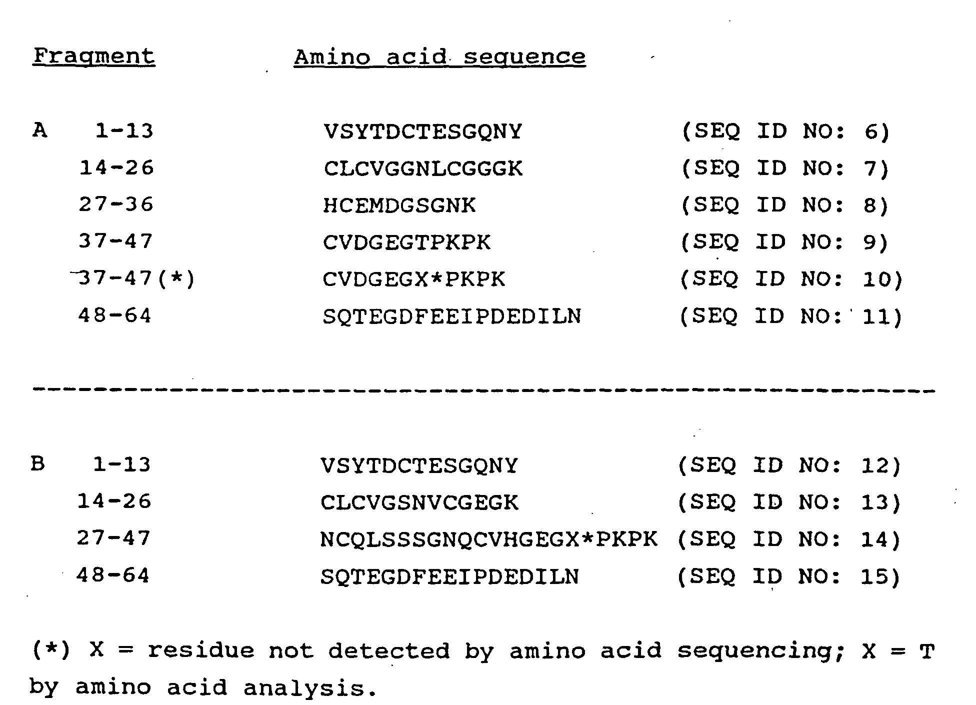

The complete amino acid sequence of P1 and P2 are therefore:

### Example 3: Chemical synthesis of the gene coding for the analogue P2₁₋₆₃-Val-Gly-OH

As already said, the analogue P2₁₋₆₃-Val-Gly-OH is one of formula (I) wherein P is the antithrombin polypeptide P2, Xaa is Val and Z is Gly-OH.

The nucleotide sequence coding for the analogue P2₁₋₆₃-Val-Gly-OH was designed on the basis of the Escherichia coli preferred codons¹¹. Moreover, a BalI restriction site was engineered very close to the 5' end of the synthetic P2₁₋₆₃-Val-Gly-OH coding sequence to allow insertion of such sequence in different expression vectors. Indeed, the same synthetic gene was used for expression of recombinant P2₁₋₆₃-Val-Gly-OH protein in bacterial and insect cells. All plasmid DNA manipulations were carried out as described by Maniatis et al¹². In the case of insect cells methods were developed which yielded protein P2₁₋₆₃-Val-Gly-OH as a secreted or cytoplasmic product. In the case of bacterial cells, methods were developed to obtain secretion to the periplasm of the recombinant product. In both cases, when a secreted product is to be obtained, it is necessary to synthesize the P2₁₋₆₃Val-Gly-OH molecule in the form of a pre-protein.
More particularly, an amino acid sequence named "leader peptide", responsible for an efficient secretion must be present at the NH₂ end of P2₁₋₆₃-Val-Gly-OH ¹³⁻¹⁴. This extra sequence is then cleaved off, in vivo, during secretion, by a specific leader peptidase, yielding the correct mature sequence¹⁵.

In the insect cells expression system, we used the leader peptide of the Vescicular Stomatitis Virus (VSV) G protein, as will be described in detail in Example 5. In the case of E. coli many examples of secretion systems have been described in the literature¹⁶⁻¹⁷. Among them, we have selected the system based on the secretion signal of the Outer Membrane Protein of E. coli (Omp A) previously published^{18.}

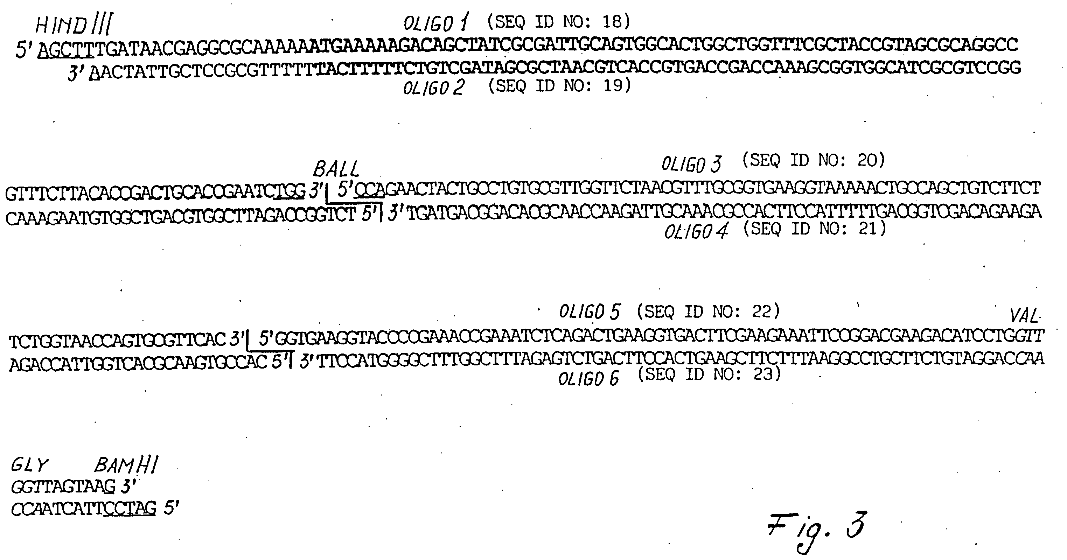

In order to prepare a suitable source of the P2₁₋₆₃-Val-Gly-OH gene for the expression vectors used in the Examples, six synthetic complementary oligonucleotides were synthetized using an automated DNA synthetizer (Applied Biosystems) and their sequence is shown in Figure 3.
We designed the first two complementary oligonucleotides coding for the OmpA leader peptide preceded by the OmpA Shine-Dalgarno sequence known to be responsible for an efficient translation of the messenger RNA¹⁹.

Their sequence, shown in Fig. 3 as oligos 1 and 2, includes also the beginning of the P2₁₋₆₃-Val-Gly-OH gene coding for the first 10 amino acids.

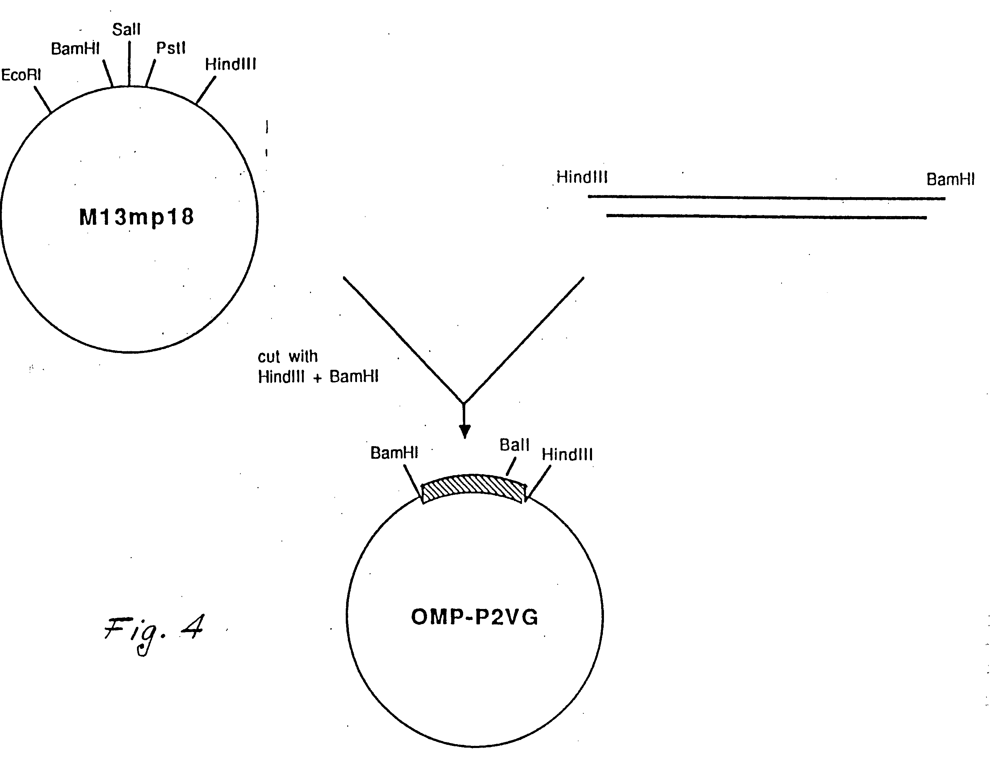

Following enzymatic phosphorylation the six oligos were assembled using DNA ligase and the resulting double-strand sequence was inserted in the M13 phage vector mp18, (Yanisch-Perron et al., Gene 33, 103-119, 1985) obtaining the recombinant plasmid OMP-P2VG which is shown in Figure 4. In order to enable insertion of the P2₁₋₆₃-Val-Gly-OH gene in the M13 vector, HindIII and BamHI sites were also added in the synthetic oligos. The correct nucleotide sequence has been verified by the Sanger method carried out on the single strand phage DNA²⁰.

### Example 4: Expression and secretion of P2₁₋₆₃-Val-Gly-OH from E. Coli cells

From OMP-P2VG the P2₁₋₆₃-Val-Gly-OH gene can be excised as a HindIII-BamHI fragment which codes for the OmpA Shine-Dalgarno and leader peptide followed by the P2₁₋₆₃-Val-Gly-OH coding sequence. This restriction fragment is now ready to be inserted in an appropriate expression vector. Several expression systems could, theoretically, be employed to obtain high level production of heterologous proteins in bacteria. The system based on the promoter Pₜᵣₚ has been used with success in our laboratory in the past¹⁹. Again, even in the case of the selected promoter, the levels of expression of a given polypeptide cannot be predicted.

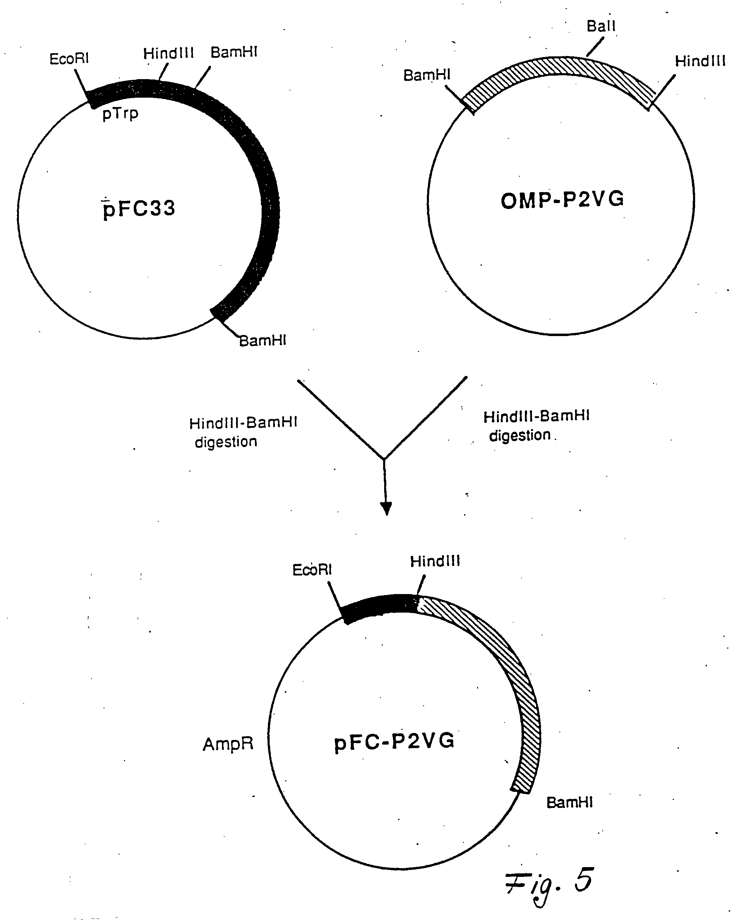

Plasmid pFC33, shown in Figure 5, has already been described in the literature¹⁹. It carries the resistance to the antibiotic ampicillin and the bacterial promoter Pₜᵣₚ which drives expression of proapolipoprotein A1. Following digestion of pFC33 with HindIII and BamHI, the large HindIII-BamHI fragment, carrying the antibiotic resistance gene and the promoter, was isolated and joined to the HindIII-BamHI fragment from OMP-P2VG coding for the P2₁₋₆₃-Val-Gly-OH gene. The details of this construction are shown in Figure 5. We isolated a new plasmid, named pFC-P2VG, which is the final plasmid for the production of P2₁₋₆₃-Val-Gly-OH in E. coli.

An object of the present invention is the use of E. coli strains of the type B for the expression and secretion to the periplasm of P2₁₋₆₃-Val-Gly-OH and the other anti-thrombin analogues of the invention. Indeed, we have found that insertion of plasmid pFC-P2VG in type B strains of the bacterium E. coli brings high level production of P2₁₋₆₃-Val-Gly-OH. Interestingly, different strain types of E. coli do not work as efficiently and it seems, therefore, that the host strain type is crucial for the successful production of P2₁₋₆₃-Val-Gly-OH.

Several type B strains of E. coli are available and can be used for the production of P2₁₋₆₃-Val-Gly-OH. Preferred strains are ATCC 12407, ATCC 11303, NCTC 10537. Below is an example of transformation of strain NCTC 10537 with plasmid pFC-P2VG and subsequent cultivation of the transformant. Competent cells of strain NCTC 10537 were prepared using the calcium chloride procedure of Mandel and Higa²¹.
Approximately 200*µ*l of a preparation of these cells at 1 x 10⁹ cells per milliliter were transformed with 2µl of plasmid DNA (approximate concentration 5µg/ml). Transformants were selected on plates of L-agar containing 100µg/ml ampicillin. Two small colonies were streaked with wooden tooth picks (each as three streaks
about 1 cm long) onto L-agar containing the same antibiotic. After 12 hours incubation at 37°C, portions of the streaks were tested for P2₁₋₆₃-Val-Gly-OH protein production by inoculation onto 10 ml of LB medium (containing ampicillin at a concentration of 150µg/ml) and incubated overnight at 37°C. The following day the cultures were diluted 1:100 in M9 medium, containing the same concentration of ampicillin, and incubated for 6 hours at 37°C.

20 ml of such culture were centrifuged at 12000xg, 4°C, for 10 minutes. The bacterial pellet was resuspended in 2 ml of 33 mM HCl Tris pH 8; an equal volume of a second solution 33 mM EDTA, 40% sucrose was then added and the total mixture was incubated under mild shaking conditions at 37°C for 10 minutes. Following centrifugation, the permeabilized cells were resuspended in 2 ml of cold water and left for 10 minutes in ice. The resulting supernatant was isolated by centrifugation and represents the periplasmic fraction of the bacterial cell.

Using a chromogenic assay that is based on the inhibition of the thrombin ability to cleave a synthetic substrate S-2238²², we have measured the presence of anti-thrombin activity in the periplasmic fraction of P2₁₋₆₃-Val-Gly-OH producing cells but not in control periplasmic fractions.

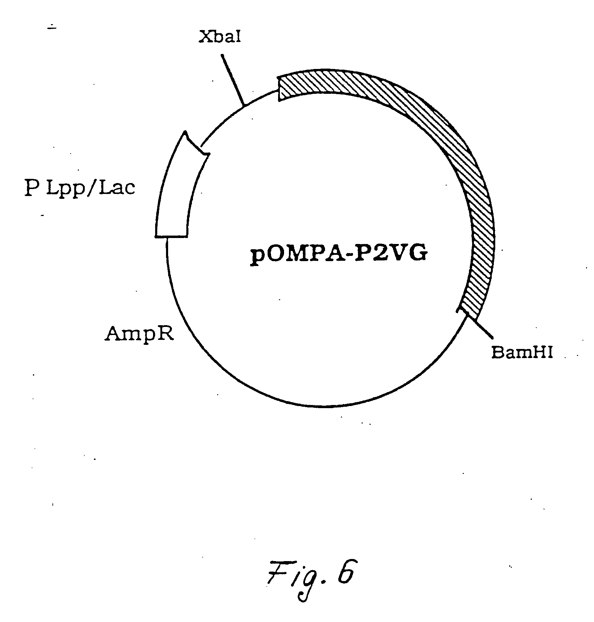

With the similar approach we have also constructed a new expression/secretion plasmid for P2₁₋₆₃-Val-Gly-OH where the promoter P_{lpp/lac}¹⁷ is present instead of the promoter Pₜᵣₚ. This different plasmid, named pOMPA-P2VG, is shown in Figure 6. Following insertion of this plasmid in E. coli strains of the type B, high levels of active P2₁₋₆₃-Val-Gly-OH were also obtained. As starting plasmid for the construction of pOMPA-P2VG we used the plasmid pIN-III-ompA3 described by Ghrayb et al²³. Conditions for cultivation and induction of expression with isopropyl-β-D-thiogalactopyranoside (IPTG) were as previously described²³.

### Example 5: Expression and secretion of protein P2₁₋₆₃-Val-Gly-OH from insect cells

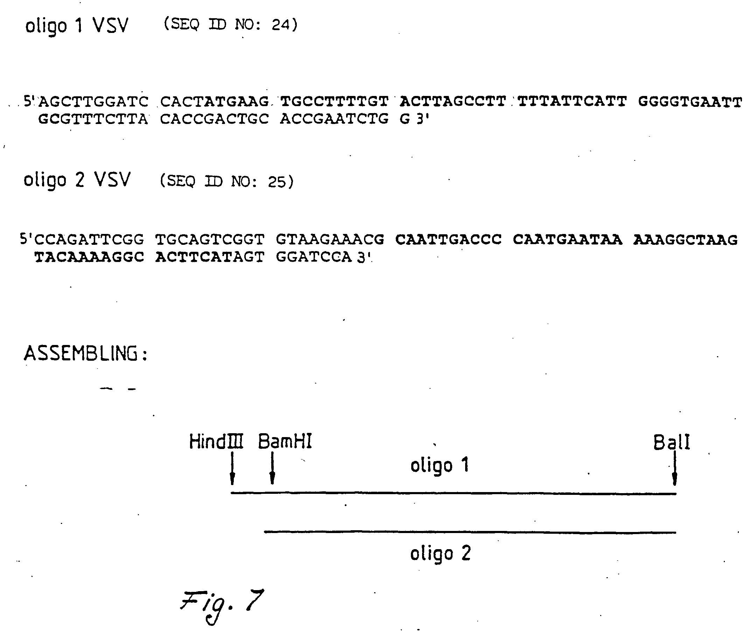

To obtain secretion of protein P2₁₋₆₃-val-Gly-OH from recombinant insect cells we had to join the P2₁₋₆₃-Val-Gly-OH coding sequence to a leader peptide that is efficiently recognized by these cells. We have used the leader peptide of the Vescicular Stomatitis Virus (VSV) G protein²⁴. Similarly to what is described above, a synthetic DNA sequence coding for the VSVG protein leader peptide followed by the beginning of the P2₁₋₆₃-Val-Gly-OH gene has been prepared and the nucleotide sequence is given in Figure 7. Also in this case we provided convenient restriction sites (HindIII, BamHI and Ball) to allow joining to the rest of the P2₁₋₆₃-Val-Gly-OH gene and to the expression vector.

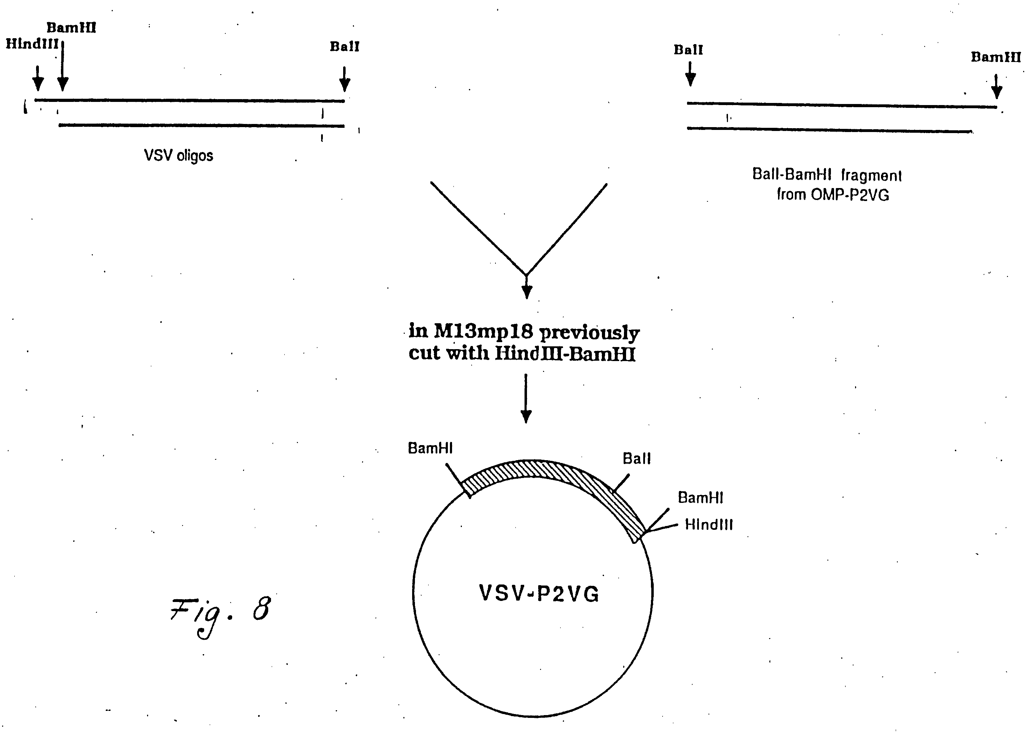

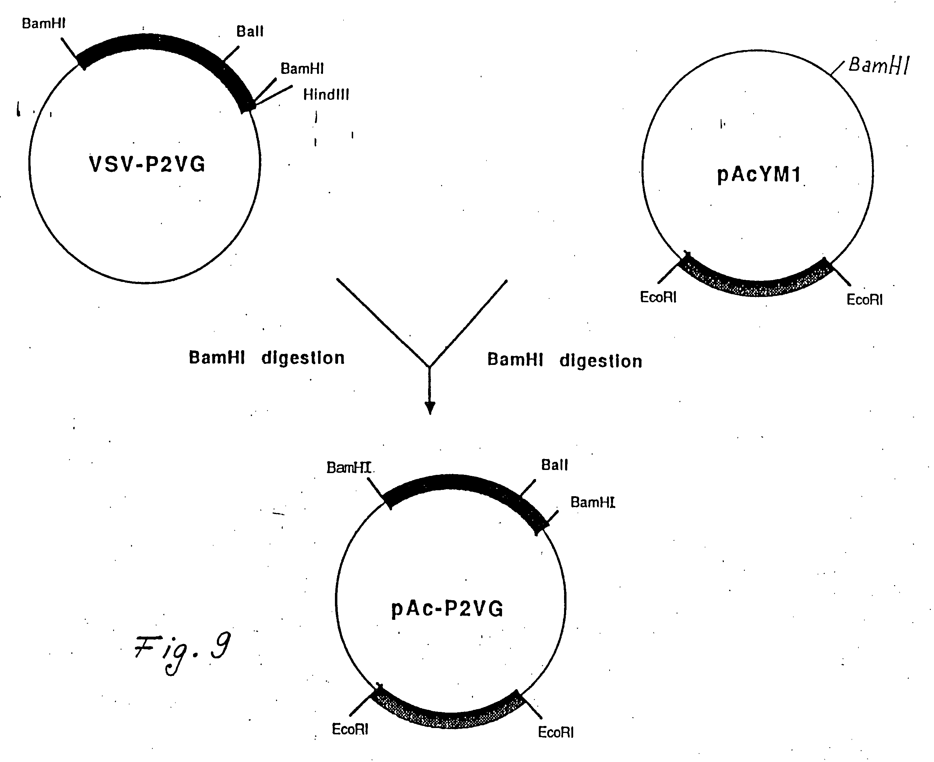

The synthetic HindIII-BalI fragment was joined to a purified BalI-BamHI fragment from OMP-P2VG carrying the P2₁₋₆₃-Val-Gly-OH gene and inserted in M13mp18 previously cut with HindIII and BamHI. This construction which yielded a new plasmid named VSV-P2VG is schematically shown in Figure 8. From VSV-P2VG we have excised a BamHI-BamHI DNA fragment carrying the P2₁₋₆₃-Val-Gly-OH gene fused to the VSV leader peptide which was then inserted into the vector pAcYMl²⁵, as shown in Figure 9. The resulting plasmid was named pAc-P2VG.

To obtain expression in insect cells, the VSV-P2VG coding sequence must be transferred to the baculovirus genome under the transcriptional control of the polyhedrin promoter. For this purpose, we co-transfected insect cells with a wild-type baculovirus DNA and with the transfer vector pAc-P2VG. As insect cells, Spodoptera frugiperda cells were chosen as host cells. Experimental details are as follows:

S. frugiperda cells were transfected with a mixture of infectious AcNPV DNA and plasmid DNA representing the individual recombinant transfer vectors by a modification of the procedure described by Summers et al²⁶. One microgram of viral DNA was mixed with 25-100 *µ*g of plasmid DNA and precipitated with (final concentrations) 0.125 M calcium chloride in the presence of 20 mM HEPES buffer, pH 7.5, 1 mM disodium hydrogen orthophosphate, 5mM potassium chloride, 140 mM sodium chloride and 10 mM glucose (total volume 1 ml).

The DNA suspension was inoculated onto a monolayer of 10⁶ S. frugiperda cells in a 35-mm tissue culture dish, allowed to adsorb to the cells for 1 h at room temperature, then replaced with 1 ml of medium. After incubation at 28°C for 3 days the supernatant fluids were harvested and used to produce plaques in S. frugiperda cell monolayers. Plaques containing recombinant virus were identified by their lack of polyhedra when examined by light microscopy. Virus from such plaques was recovered and after further plaque purification was used to produce polyhedrin-negative virus stocks.

The above procedure allowed us to isolate a recombinant baculovirus whose genome carried the P2₁₋₆₃-Val-Gly-OH gene under control of the polyhedrin promoter and of the VSV G protein leader peptide. We used this virus to infect S. frugiperda cells according to well-established procedures²⁶, at a multiplicity of infection of 10. Infected cells were then cultivated in spinner culture or in monolayers in the presence of 10% foetal calf serum according to published methods²⁶. In both conditions, the S-2238 chromogenic assay showed the presence of an anti-thrombin activity in the culture supernatants of the infected cells.

### Example 6: Expression of protein P2₁₋₆₃-Val-Gly-OH in the cytoplasm of insect cells

Protein P2₁₋₆₃-Val-Gly-OH could also be produced and accumulated in the cytoplasm of S. frugiperda cells. This approach generally gives a better yield of heterologous proteins since it utilizes the expression signals of polyhedrin which is a non-secreted viral protein.

Our approach to obtain large quantities of recombinant protein P2₁₋₆₃-Val-Gly-OH is based on the expression of a fusion polypeptide where the first 18 amino acids of polyhedrin are joined in frame to the 65 amino acids of P2₁₋₆₃-Val-Gly-OH. The presence of the NH₂ end sequence of polyhedrin allows high level expression²⁷. In addition, between the polyhedrin portion and the P2₁₋₆₃-Val-Gly-OH sequence we put a methionine residue which allows the release of the P2₁₋₆₃-Val-Gly-OH moiety by treatment of the hybrid protein with CNBr.

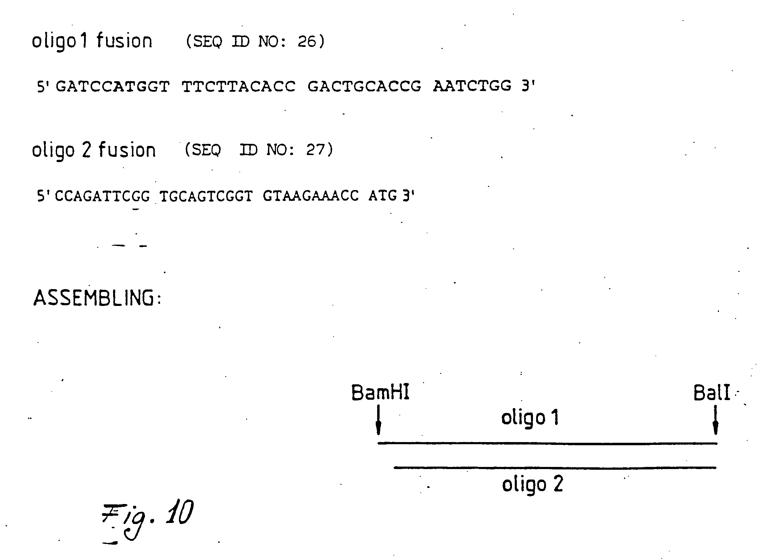

Similarly to the previous approaches, we prepared a synthetic DNA fragment which could allow the joining of the BalI-BamHI, fragment from OMP-P2VG to an appropriate transfer vector. The new synthetic piece, shown in Figure 10, includes also BamHI and BalI sites for subsequent manipulations.

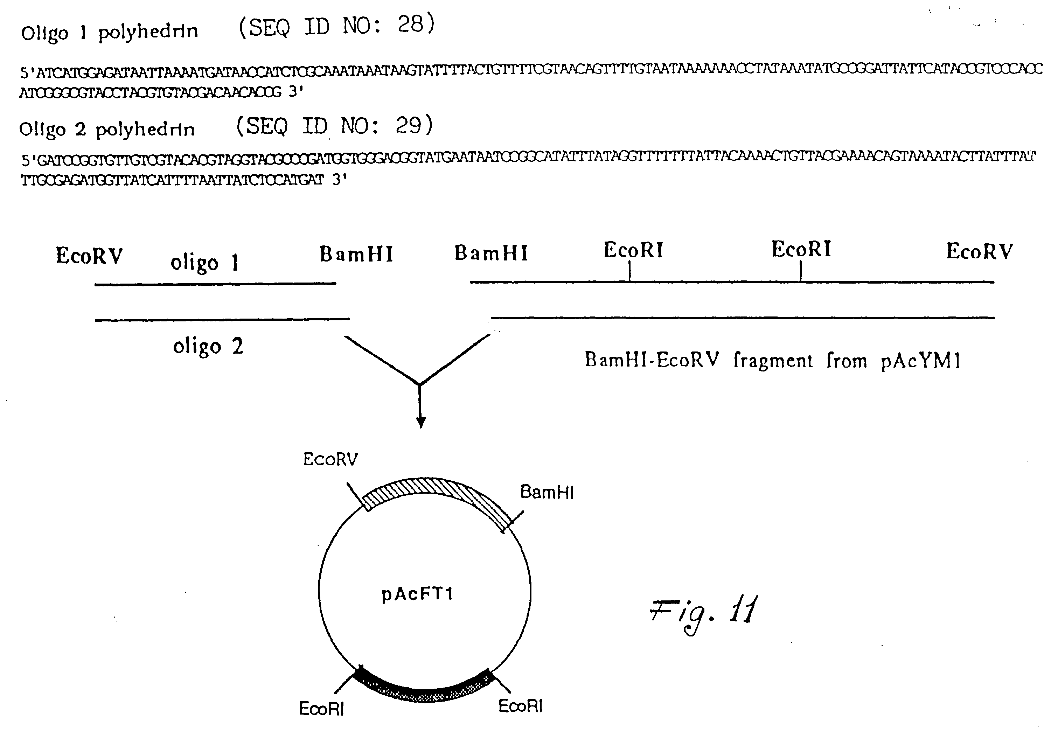

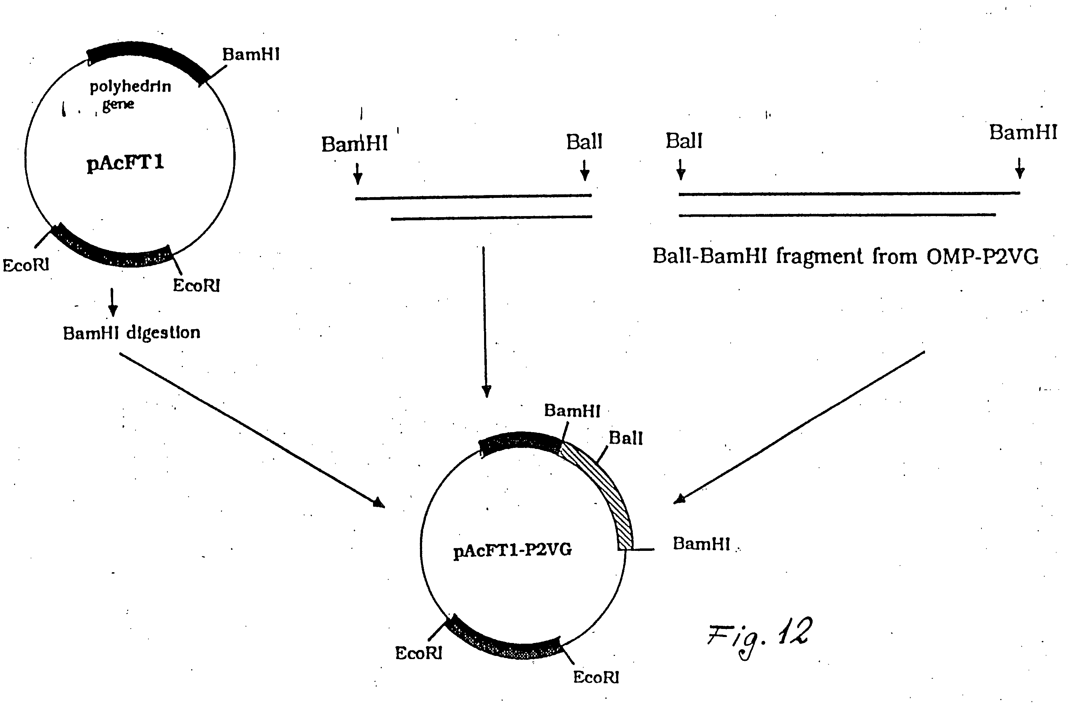

A different transfer vector, pAcFT1, carrying the nucleotide sequence coding for the first 18 amino acids of polyhedrin has been obtained (Figure 11). Briefly, the EcoRV-BamHI fragment of pAcYM1²⁵ has been replaced by a synthetic oligonucleotide containing the polyhedrin gene sequence from nucleotide -92 to nucleotide +55. A convenient BamHI site is present after this sequence and it has been used for insertion of the complete P2₁₋₆₃-Val-Gly-OH coding sequence according to a scheme illustrated in Figure 12. Through this construction, we obtained a new plasmid, named pAcFT1-P2VG, which has been used to transfer the hybrid gene to the baculovirus genome.

The recombinant baculovirus was obtained as described in Example 5. Infection of S. frugiperda cells was carried out according to standard procedures²⁶. Cultivation of infected insect cells leads to the cytoplasmic accumulation of the fusion protein. This hybrid protein was the source of recombinant protein P2₁₋₆₃-Val-Gly-OH. Several methods are available from the literature which can be used to cleave the hybrid with CNBr²⁸⁻²⁹. The application of the method of Olson et al has allowed us to obtain the correct polypeptidic sequence of P2₁₋₆₃-Val-Gly-OH. This molecule displayed anti-thrombin activity.

### Example 9

### Biological activity of P2₁₋₆₃-Val-Gly-OH

### Antithrombin activity

The antithrombin activity of the analogues P2₁₋₆₃-Val-Gly-OH of the present invention was determined on the basis of the rapid and stoechiometric reaction of the analogue peptides with thrombin.
This activity was measured quantitatively by means of titration of a standard solution of thrombin³³.
Thrombin activity was calibrated with the International Standard Preparation of Thrombin (c 70/157) obtained from the National Institute for Biological Standards and Control (London, U.K.) and expressed in National Institute of Health Units (N.I.H. units).
The thrombin neutralizing activity of the samples was expressed as antithrombin units (ATU); one ATU is the amount of test compound which neutralizes an NIH unit of thrombin. Recombinant hirudin (HV1 variant; rHVl) and recombinant P2 protein (rP2) were considered as reference compounds.
The test was performed as follows:
0.2 ml of a standard solution of 0.05% human fibrinogen (Kabi Vitrum, Sweden) in Tris HCl buffer pH 7.4 were incubated at 37°C in the presence of 0.01 to 0.1 ml of the solutions of P2₁₋₆₃-Val-Gly-OH.
Thereafter, aliquots of 0.005 ml (0.5 NIH units) of a 100 NIH/ml thrombin standard solution were added progressively each minute and mixed gently.
The end point of the titration was considered to be reached when a fibrin clot was formed within one minute.
Indeed, clot formation only occurred when a sufficient amount of thrombin was added to the reaction mixture, the added thrombin being able to neutralize the total amount of the analogue present in the mixture.
The antithrombin activity of the analogue P2₁₋₆₃-Val-Gly-OH has been expressed in ATU/mg protein (see table 1).
Protein content was determined by aminoacid analysis.

### Chromogenic substrate assay

The principle of the method is based on the inhibitory activity of P2₁₋₆₃-Val-Gly-OH on the reaction between thrombin and its specific chromogenic substrate S-2238³⁴.
Recombinant hirudin HV1 and recombinant P2 protein were used as references.
The test was performed as follows:
630 µl of a 0.5 mM solution of S-2238 in 0.05 M Tris buffer pH 8.3 containing 0.1N NaCl and 100 KIU/ml aprotinin were pre-incubated for 10 min. at 25°C. After this pre-incubation period, 70 *µ*l of a 2 IU/ml thrombin solution in 0.025 M sodium phosphate buffer pH 6.6 containing 0.3 M NaCl and 0.5% bovine albumin were added.
For the controls, 70 µl of buffer were used.
Immediately after the addition of thrombin, the absorbance variations were recorded spectrophotometrically at a wave length of 405 nm.
Recordings were made kinetically each minute over a total period of 6 minutes.
Thereafter, 20 µl of the solutions of the tested analogue were added and absorbance variations were recorded again as described before.
Final concentrations of the test compounds were 1.25, 2.5, 7.5, 10 and 15 ng/ml in 0.025 M sodium phosphate buffer pH 6.6 containing 0.3 M NaCl.
The absorbance variations per minute (△ Abs/min) were determined respectively before and after the addition of the sample and the mean Δ Abs/min. were calculated.
The inhibition of the reaction between thrombin and the chromogenic substrate S-2238 was calculated as the ratio (expressed as percentage) between the mean △ Abs/min. before and after the addition of the analogue.
A standard curve has been established on the basis of the percent inhibition versus the concentration of the sample.
Final results were expressed as final concentrations of the test compounds inducing 50% inhibition of the reaction between thrombin and its chromogenic substrate.

### The thrombin time

The antithrombin activity of the analogue P2₁₋₆₃-Val-Gly-OH has been determined by measuring the thrombin time in human plasma.
Recombinant hirudin HV1 (rHV1) and recombinant P2 protein (rP2) were used as references.
The test was performed as follows:
Mixtures of normal, human citrated 'plasma containing increasing final concentrations of the tested analogue or the reference compounds were placed in an automatic coagulometer (ACL 300 Research, Instrumentation Laboratory, Italy), and human thrombin (Fibrindex, Ortho Diagnostics, Italy: 5 IU/ml final concentration) was added.
The clotting times were recorded and plotted against the final concentration of each sample.
Final results were expressed as the final concentration of each_compound required to double the normal value of the thrombin time (ratio=2).
In all the test performed, the antithrombin activity of P2₁₋₆₃-Val-Gly-OH resulted considerably superior to that of the reference compounds.
Table 1 reports the data obtained with the analogue P2₁₋₆₃-Val-Gly-OH in the biological assays discussed above.

**TABLE 1**

| Compound | ATU/mg | S-2238 IC50 in ng/ml | Thrombin Time in *µ*g/ml |
|---|---|---|---|
| rHV1 | 11.000 | 5.47 | ratio=2 |
| | | | |
| rP2 | 11.900 | 5.43 | at 0.132 µg/ml ratio=2 |
| P2₁₋₆₃-Val-Gly-OH | 20.000 | 0.6 | at 0.131 µg/ml ratio=2 |
| | | | at 0.047 µg/ml |

### References

1) Markwardt, F. 1970, Methods in Enzymology, 19, p. 924
2) Markwardt, F. 1985, Biomed. Biochim. Acta. 44, p. 1007
3) Markwardt, F. Hauptmann, J., Nowak, G., Klessen, C., and Walsmann, P. 1982. Thromb. Haemostasis 47, p. 226
9) Lundblad, R.L., 1971, Biochemistry, 10: 2501-2506
10) Dupont D., Keim P., Chui A., Bello R. and Wilson K., Derivatizer-Analyser User Bulletin No. 1, Applied Biosystems Inc., 1987
11) Grosjeans H. and Fiers W. 1982. Gene, 18, p. 199
12) Maniatis T., Fritsch E.F. and Sambrook J. 1982. Cold Spring Harbor, NY
13) Blobel G. and Dobberstain B. 1975. J. Cell Biology, 67, p. 83
14) Pages J.M. 1983, Biochimie, 65, p. 531
15) Wolfe P.B. 1983. J. Biol. Chem. 258, p. 12073
16) Talmadge K., Stahl S. and Gilbert W. 1980. Proc. Natl. Acad. Sci. USA, 77, p. 3369
17) Oka T., Sakamoto S., Miyoshi K., Fuwa T., Yoda K., Yamasaki M., Tamura G. and Miyake K. 1985. Proc. Natl. Acad. Sci. USA, 82, p. 7212
18) Henning V., Royer H.D., Teather R.M., Hindennach I. and Hollenberg C.P. 1979. Proc. Natl. Acad. Sci. USA, 76, p. 4360
19) Isacchi A., Sarmientos P., Lorenzetti R. and Soria M. 1989, Gene 81, p. 129
20) Sanger, F., Nicklen, S., and Coulson, A.R. 1977, Proc. Natl. Acad. Sci. USA 74, p. 5463.
21) Mandel M. and Higa A.J. 1970. J. Mol. Biology, 53, p.154
22) Krstenansky, J.K., and Mao, S.J.T. 1987. FEBS Lett. 211, p. 10
23) Ghrayeb J., Kimura H., Takahara M., Hsiung H., Masui Y. and Inouye M. 1984. EMBO Journal 3, p. 2437
24) Bailey, M.J., McLeod, D.A., Kang, C., and Bishop, D.H.L. 1989. Virology 169, p. 323
25) Matsuura, Y., Possee, R.D., Overton, H.A. and Bishop. D.H.L. 1987. J. Gen. Virol. 68, p. 1233
26) Summers, M.D., and Smith, G.E. 1987, Texas Agricultural Experiment Station Bulletin No. 1555
27) Luckow, V.A. and Summers, M.D. 1988, Virology, 167, p.56
28) Gross E. 1967. Methods in Enzymology, 11, p. 238
29) Olson H., Lind P., Pohl G., Henrichson C., Mutt V., Jornvall H., Josephson S., Uhlen M. and Lake M. 1987, Peptides, 9, p. 301
33) Lundblad R.L., et al., Methods in Enzymology, 45, 156-161,-1976).
34) Wiman et al., BBA, 579, 142-154, 1979

## Claims

1. A polypeptide comprising a sequence represented by formula (I) wherein P2₁₋₆₃ is the amino acid sequence from position 1 to position 63 of the polypeptide P2 (SEQ ID NO: 2), and a pharmaceutically acceptable salt thereof.

2. A polypeptide according to claim 1 in which the amino acid sequence represented by formula (I) is preceded by a Met residue.

3. A polypeptide according to claim 1 in which the amino acid sequence represented by formula (I) is preceded by all or part of a leader sequence.

4. A polypeptide according to claim 3, in which all or part of the following leader sequence is present:

5. A polypeptide according to claim 1, which is a fusion protein in which the amino acid sequence represented by formula (I) is fused to a carrier sequence.

6. A process for the preparation of a polypeptide as defined in any one of the preceding claims or a pharmaceutically acceptable salt thereof, which process comprises:
(a) providing a non-human host, transformed with an expression vector comprising a DNA sequence encoding a said polypeptide, under such conditions that the said polypeptide is expressed; and
(b) isolating the said polypeptide thus obtaining a polypeptide as defined in any one of the precedings claims or a pharmaceutically acceptable salt thereof.

7. A process according to claim 6, wherein the non-human host is a bacterium, yeast, mammalian cell line, insect cell line or non-human animal.

8. A process according to claim 7, wherein the host is a strain of E. coli type B or a Spodoptera frugiperda cell line.

9. A process for the preparation of a polypeptide as defined in any one of claims 1 to 5 or a pharmaceutically acceptable salt thereof, which process comprises:
(a) chemically synthesising the said polypetide; and
(b) isolating the said polypeptide thus obtained or a pharmaceutically acceptable salt thereof.

10. A process according to claim 9, wherein step (a) is effected by solid-phase synthesis.

11. A pharmaceutical composition comprising a pharmaceutically acceptable carrier or diluent and, as active ingredient, a polypeptide as defined in any one of claims 1 to 5 or a pharmaceutically acceptable salt thereof.

12. An expression vector comprising a DNA sequence encoding a polypeptide as defined in any one of claims 1 to 5.

13. A vector according to claim 12, which is a plasmid.

14. A vector according to claim 12, which is a virus.

15. A vector according to claim 14, wherein the virus is a recombinant baculovirus in which the polyhedrin promoter is operably linked to the said DNA sequence.

16. A vector according to any one of claims 12 to 15, wherein the said DNA sequence further encodes a leader peptide capable of directing secretion of the said polypeptide from cells in which the said polypeptide is expressed.

17. A vector according to any one of claims 12 to 16, wherein the said DNA sequence encodes a fusion protein which is cleavable to release a polypeptide of formula (I) as defined in claim 1.

18. A non-human host transformed with a compatible expression vector according to any one of claims 12 to 17.

19. A non-human host according to claim 18, which is a bacterium, yeast, mammalian cell line, insect cell line or non-human animal.

20. A host according to claim 19, which is a strain of E. coli type B or a Spodoptera frugiperda cell line.

21. A DNA sequence encoding a polypeptide as defined in any one of claims 1 to 5 .

22. A DNA sequence according to claim 21, which is a synthetic DNA sequence.

23. A DNA sequence according to claim 21, which is a mutagenized cDNA.

24. A DNA sequence according to any one of claims 21 to 23, which further encodes a leader peptide capable of directing secretion of the said polypeptide from cells in which the said polypeptide is expressed.

25. A DNA sequence according to any one of claims 21 to 24, which encodes a fusion protein which is cleavable to release a polypeptide of formula (I) as defined in claim 1.

26. A process for the preparation of a non-human host in which a polypeptide as defined in any one of claims 1 to 5 is able to be expressed, which process comprises transforming a non-human host with a compatible expression vector according to any one of claims 12 to 17.

27. A process according to claim 26, wherein the said expression vector has been prepared by:
(a) chemically synthesising a DNA sequence encoding the said polypeptide; and
(b) inserting the said DNA sequence into an expression vector.

28. A process according to claim 26, wherein the said expression vector has been prepared by:
(a) producing and isolating a cDNA encoding the polypeptide P2 (SEQ ID NO: 2) from mRNA from a leech of the species Hirudinaria manillensis;
(b) mutagenising the isolated cDNA so as to obtain a nucleotide sequence encoding a polypeptide as defined in any one of claims 1 to 5; and
(c) inserting the said nucleotide sequence into an expression vector.

29. A DNA sequence according to claim 21, which consists essentially of the sequence depicted in SEQ ID NO: 16.

## Patentansprüche

1. Polypeptid, umfassend eine Sequenz der Formel (I) worin P2₁₋₆₃ die Aminosäuresequenz von Position 1 bis Position 63 des Polypeptids P2 (SEQ ID NO: 2) ist, und ein pharmazeutisch verträgliches Salz davon.

2. Polypeptid nach Anspruch 1, worin der Aminosäuresequenz der Formel (I) ein Met-Rest vorausgeht.

3. Polypeptid nach Anspruch 1, worin der Aminosäuresequenz der Formel (I) die Gesamtheit oder ein Teil einer einer Leadersequenz vorausgeht.

4. Polypeptid nach Anspruch 3, worin die Gesamtheit oder ein Teil der folgenden Leadersequenz vorhanden ist:

5. Polypeptid nach Anspruch 1, das ein Fusionsprotein ist, worin die Aminosäuresequenz der Formel (I) an eine Trägersequenz fusioniert ist.

6. Verfahren zur Herstellung eines Polypeptids wie in einem der vorstehenden Ansprüche definiert, oder eines pharmazeutisch verträglichen Salzes davon, umfassend:
(a) Bereitstellen eines nicht-menschlichen Wirts, der mit einem Expressionsvektor transformiert ist, der eine DNA-Sequenz, die das Polypeptid codiert, umfasst unter solchen Bedingungen, dass das Polypeptid exprimiert wird, und
(b) Isolieren des Polypeptids, wodurch ein Polypeptid wie in einem der vorstehenden Ansprüche definiert, oder ein pharmazeutisch verträgliches Salz davon erhalten wird.

7. Verfahren nach Anspruch 6, worin der nicht-menschliche Wirt ein Bakterium, Hefe, eine Säugerzelllinie, eine Insektenzelllinie oder ein nicht-menschliches Tier ist.

8. Verfahren nach Anspruch 7, worin der Wirt ein Stamm der E. coli Typ B- oder einer Spodoptera frugiperda-Zelllinie ist.

9. Verfahren zur Herstellung eines Polypeptids, wie in einem der Ansprüche 1 bis 5 definiert, oder eines pharmazeutisch verträglichen Salzes davon, wobei man
(a) das Polypeptid chemisch synthetisiert, und
(b) das so erhaltene Polypeptid oder ein pharmazeutisch verträgliches Salz davon isoliert.

10. Verfahren nach Anspruch 9, wobei Stufe (a) mittels Festphasensynthese durchgeführt wird.

11. Pharmazeutisches Präparat, umfassend einen pharmazeutisch verträglichen Träger oder ein Verdünnungsmittel und als Wirkstoff ein Polypeptid, wie in einem der Ansprüche 1 bis 5 definiert, oder ein pharmazeutisch verträgliches Salz davon.

12. Expressionsvektor, umfassend eine DNA-Sequenz, die ein Polypeptid wie in einem der Ansprüche 1 bis 5 definiert codiert.

13. Vektor nach Anspruch 12, der ein Plasmid ist.

14. Vektor nach Anspruch 12, der ein Virus ist.

15. Vektor nach Anspruch 14, wobei das Virus ein rekombinantes Baculovirus ist, in dem der Polyhedrinpromotor funktionell mit der DNA-Sequenz verknüpft ist.

16. Vektor nach einem der Ansprüche 12 bis 15, wobei die DNA-Sequenz ferner ein Leaderpeptid codiert, das die Sekretion des Polypeptids aus Zellen, in denen das Polypeptid exprimiert wird, steuern kann.

17. Vektor nach einem der Ansprüche 12 bis 16, wobei die DNA-Sequenz ein Fusionsprotein codiert, das spaltbar ist, wobei ein Polypeptid der Formel (I) wie in Anspruch 1 definiert freigesetzt wird.

18. Nicht-menschlicher Wirt, transformiert mit einem kompatiblen Expressionsvektor nach einem der Ansprüche 12 bis 17.

19. Nicht-menschlicher Wirt nach Anspruch 18, der ein Bakterium, Hefe, eine Säugerzelllinie, eine Insektenzelllinie oder ein nicht-menschliches Tier ist.

20. Wirt nach Anspruch 19, der ein Stamm der E. coli Typ B- oder einer Spodoptera frugiperda-Zelllinie ist.

21. DNA-Sequenz, die ein Polypeptid wie in einem der Ansprüche 1 bis 5 definiert codiert.

22. DNA-Sequenz nach Anspruch 21, die eine synthetische DNA-Sequenz ist.

23. DNA-Sequenz nach Anspruch 21, die eine mutierte cDNA ist.

24. DNA-Sequenz nach einem der Ansprüche 21 bis 23, die ferner ein Leaderpeptid codiert, das die Sekretion des Polypeptids aus Zellen, in denen das Polypeptid exprimiert wird, steuern kann.

25. DNA-Sequenz nach einem der Ansprüche 21 bis 24, die ein Fusionsprotein codiert, das spaltbar ist, so dass ein Polypeptid der Formel (I) wie in Anspruch 1 definiert freigesetzt wird.

26. Verfahren zur Herstellung eines nicht-menschlichen Wirts, in dem ein Polypeptid wie in einem der Ansprüche 1 bis 5 definiert exprimiert werden kann, wobei man einen nicht-menschlichen Wirt mit einem kompatiblen Expressionsvektor nach einem der Ansprüche 12 bis 17 transformiert.

27. Verfahren nach Anspruch 26, wobei der Expressionsvektor dadurch hergestellt wurde, dass:
(a) chemisch eine DNA-Sequenz, die das Polypeptid codiert, synthetisiert wurde, und
(b) die DNA-Sequenz in einen Expressionsvektor insertiert wurde.

28. Verfahren nach Anspruch 26, wobei der Expressionsvektor dadurch hergestellt wurde, dass:
(a) eine cDNA, die das Polypeptid P2 (SEQ ID NO: 2) codiert, aus mRNA aus einem Blutegel der Spezies Hirudinaria manillensis produziert und isoliert wurde;
(b) die isolierte cDNA so mutiert wurde, dass eine Nukleotidsequenz erhalten wurde, die ein Polypeptid wie in einem der Ansprüche 1 bis 5 definiert codiert, und
(c) die Nukleotidsequenz in einen Expressionsvektor insertiert wurde.

29. DNA-Sequenz nach Anspruch 21, die im Wesentlichen aus der in SEQ ID NO: 16 dargestellten Sequenz besteht.

## Revendications

1. Un polypeptide comprenant une séquence représentée par la formule (I) dans laquelle P2₁₋₆₃ est une séquence d'acide amino de la position 1 à la position 63 du polypeptide P2 (SEQ ID NO : 2), et en conséquence un sel pharmaceutique approprié.

2. Un polypeptide selon la revendication 1,
dans lequel la séquence d'acide amino, représenté par la formule (I), est précédée par un résidu Met.

3. Un polypeptide selon la revendication 1,
dans lequel la séquence d'acide amino, représenté par la formule (I), est précédée par tout, ou partie, d'une séquence leader.

4. Un polypeptide selon la revendication 3,
dans lequel tout, ou partie, de la séquence leader suivante est présente :

5. Un polypeptide selon la revendication 1,
qui est une protéine de fusion dans laquelle la séquence d'acide amino représentée par la formule (I) est unie à une séquence porteuse.

6. Un procédé pour la préparation d'un polypeptide comme défini dans l'une des revendications précédentes, ou d'un sel pharmaceutique approprié de celui-ci, ce procédé comprenant :
(a) comprenant une séquence d'ADN encodant ledit polypeptide, dans les conditions où ledit polypeptide est obtenu ; et
(b) l'isolement dudit polypeptide afin d'obtenir un polypeptide, comme défini dans l'une des revendications précédentes, ou un sel pharmaceutique approprié de celui-ci.

7. Un procédé selon la revendication 6,
dans lequel l'hôte non-humain est une bactérie, une levure, une ligne de cellule de mammifère, une ligne de cellule d'insecte ou d'animal non-humain.

8. Un procédé selon la revendication 7,
dans lequel l'hôte est une souche de type B E. coli ou une ligne de cellule frugiperda Spodoptera.

9. Un procédé pour la préparation d'un polypeptide, comme défini selon une des revendications 1 à 5 ou, d'un sel pharmaceutique approprié de celui-ci, ce procédé comprenant :
(a) une synthèse chimique dudit polypeptide ;et
(b) un isolement dudit polypeptide ainsi obtenu ou d'un sel pharmaceutique approprié de celui-ci.

10. Un procédé selon la revendication 9,
dans lequel l'étape (a) est effectuée par une synthèse en phase solide.

11. Une composition pharmaceutique comprenant une solution, ou un diluant, pharmaceutique appropriée et, comme ingrédient actif, un polypeptide comme défini selon une des revendications 1 à 5 ou, un sel pharmaceutique approprié de celui-ci.

12. Un vecteur d'expression comprenant une séquence d'ADN encodant un polypeptide comme défini selon l'une des revendications 1 à 5.

13. Un vecteur selon la revendication 12,
qui est un plasmide.

14. Un vecteur selon la revendication 12,
qui est un virus.

15. Un vecteur selon la revendication 14,
où le virus est un baculovirus recombinant dans lequel l'instigateur polyhédrine est lié de façon opérable à ladite séquence d'ADN.

16. Un vecteur selon l'une des revendications 12 à 15,
où ladite séquence d'ADN encode, en outre, un peptide leader capable de diriger les sécrétions dudit polypeptide, depuis les cellules dans lesquelles ledit polypeptide est expédié.

17. Un vecteur selon l'une des revendications 12 à 16,
où ladite séquence d'ADN encode une protéine de fusion qui est sécable afin de libérer un polypeptide de formule (I), tel que défini dans la revendication 1.

18. Un hôte non-humain transformé avec un vecteur d'expression compatible selon l'une des revendications 12 à 17.

19. Un hôte non-humain selon la revendication 18,
qui est une bactérie, une levure, une ligne de cellule de mammifère, une ligne de cellule d'insecte ou d'animal non-humain.

20. Un hôte selon la revendication 19,
qui est une souche de type B E. coli ou une ligne de cellule frugiperda Spodoptera.

21. Une séquence d'ADN encodant un polypeptide tel que défini dans l'une des revendications 1 à 5.

22. Une séquence d'ADN selon la revendication 21, qui est une séquence d'ADN synthétique.

23. Une séquence d'ADN selon la revendication 21,
qui est un ADNc génétiquement manipulé.

24. Une séquence d'ADN selon l'une des revendications 21 à 23,
qui encode, en outre, un peptide leader capable de diriger les sécrétions dudit polypeptide depuis les cellules dans lesquelles ledit polypeptide est exprimé.

25. Une séquence d'ADN selon l'une des revendications 21 à 24,
qui encode une protéine de fusion, qui est sécable, afin de libérer un polypeptide de formule (I) comme défini selon la revendication 1.

26. Un procédé pour la préparation d'un hôte non-humain dans lequel un polypeptide comme défini selon l'une des revendications 1 à 5 est capable d'être exprimé,
ce procédé comprenant la transformation d'un hôte non-humain avec un vecteur d'expression compatible selon l'une des revendications 12 à 17.

27. Un procédé selon la revendication 26,
dans lequel ledit vecteur d'expression a été préparé par :
(a) une synthèse chimique d'une séquence d'ADN encodant ledit polypeptide ; et
(b) l'insertion de ladite séquence d'ADN dans un vecteur d'expression.

28. Un procédé selon la revendication 26,
dans lequel le vecteur d'expression a été préparé par :
(a) la production et l'isolement d'un ADNc encodant le polypeptide P2 (SEQ ID NO : 2) depuis l'ARNm d'un parasite de l'espèce Hirudinaria manillensis ;
(b) la manipulation génétique de l'ADNc isolé afin d'obtenir une séquence nucléotide encodant un polypeptide comme défini selon l'une des revendications 1 à 5 ; et
(c) l'insertion de ladite séquence nucléotide dans un vecteur d'expression.

29. Une séquence d'ADN selon la revendication 21, qui consiste essentiellement en la séquence décrite dans SEQ ID NO: 16.
